# EUROPEAN PATENT APPLICATION

(11) **EP 2 065 463 A2**
(43) Date of publication of application: **03.06.2009**
(21) Application number: 08022518.8
(22) Date of filing: 05.04.2007
(51) Int. Cl.: C12N 5/06, A61P 25/02, A61K 35/30

(54) **Survival and development of neural cells**

(30) Priority: 07.04.2006 US 790191 P
(62) Divisional of application: 07789637.1
(71) Applicant: Neuro Therapeutics AB, 171 77 Stockholm (SE)
(72) Inventor: Arenas, Ernesto, 17 177 Stockholm (SE); Mira, Helena, 46100 València (ES); Edman, Linda, 17 177 Stockholm (SE); Sousa, Kyle, Ann Arbor, MI 48109-0622 (US)
(74) Representative: Walton, Seán Malcolm

(57) **Abstract**

Promoting survival, maturation and/or differentiation of a stem or neural cell, by means of modulating activity of a protein encoded by a Nurr1 up-regulated gene or a Nurr1 downregulated gene in the cell.

## Description

The present invention relates to materials and methods for promoting survival, differentiation, maturation and/or acquisition of a region-specific neuronal cell fate, in stem cells, neural stem, progenitor or precursor cells, and dopaminergic neurones, and methods for identifying substances suitable for use in such methods.

Parkinson's Disease (PD), the second most common human neurodegenerative disorder, primarily results from selective and progressive degeneration of midbrain dopamine-synthesizing neurons. Previously, human foetal mesencephalic tissue has been grafted into Parkinsonian patients with positive results, but there are practical and ethical difficulties with such approaches. In particular, implanted non-autologous tissue has a limited viability and may be rejected by the immune system. In addition, each foetus provides only a small number of cells.

Cell-replacement strategies, based on transplantation of stem cells manipulated to generate functional dopaminergic (DA) neurons, have recently raised hope for the treatment of PD patients. Stem, progenitor or precursor cells are an ideal material for transplantation therapy since they can be expanded and instructed to assume a specific neuronal phenotype. These cells would circumvent ethical and practical issues surrounding the use of human foetal tissue for transplantation. Another application of these cells is drug testing and toxicity screening of molecules to be used in human disease. Human stem cells guided to differentiate into dopaminergic neurons could be used for testing drugs for desired and/or adverse effects. In particular, dopaminergic neurons would be an ideal tool to test drugs that work on dopaminergic neurons or to be used in diseases affecting dopaminergic neurons such as schizophrenia, depression, Parkinson's disease, and other movement disorders.

Embryonic, neural and multipotent stem cells have the ability to differentiate into neural cell lineages including neurons, astrocytes and oligodendrocytes. Moreover, stem cells can be isolated, expanded, and used as source material for brain transplants (Snyder et al., 1992; Rosenthal, 1998; Bain et al., 1995; Gage, et al. 1995; Okabe et al., 1996; Weiss, et al., 1996; Snyder, et al. 1996; Martinez-Serrano, et al. 1997; McKay, 1997; Deacon et al., 1998; Studer, et al. 1998; Bjorklund and Lindvall 2000; Brustle et al., 1999; Lee et al., 2000; Shuldiner et al., 2000 and 2001; Reubinoff et al., 2000 and 2001; Tropepe et al., 2001; Zhang et al., 2001; Price and Williams 2001; Arenas 2002; Bjorklund et al., 2002; Rossi and Cattaneo, 2002; Gottlieb et al., 2002).

Genetic engineering of mouse neural stem cells and embryonic stem (ES) cells using the Nuclear receptor related-1 (Nurr1) has proven successful for generating cell populations enriched in DA neurons (Wagner et al., 1999; Chung et al., 2002; Kim et al., 2002).

Nurr1 is a transcription factor of the thyroid hormone/retinoic acid nuclear receptor superfamily that is highly expressed in the developing and adult ventral midbrain (VM) (Zetterström et al., 1996a). In this brain region, the transcription factor Nurr1 is expressed at E10.5 by dopaminergic cell precursors, this is, prior to the birth of tyrosine hydroxylase positive (TH+) neurons *in vivo*, and is required for proper dopaminergic neuronal differentiation (Zetterström et al., 1997; Saucedo-Cardenas et al., 1998; Castillo et al., 1998). Moreover, in the Nurr1-null mice, midbrain TH+ neurons are not born and in that area cells die by apoptosis at embryonic day 18.5 (Zetterström et al., 1997; Wallén et al., 1999). Increased loss of DA neurons is observed in Nurr1^{+/-} mice (Le et al., 1999).

We previously demonstrated that Nurr1 overexpression induces neuronal differentiation of the c17.2 neural stem cell line and that these cells adopt a dopaminergic fate upon co-culture with embryonic/newborn midbrain astrocytes (Wagner et al., 1999 and WO00/66713). The majority of these committed neurons adopt a dopaminergic fate upon co-culture with embryonic/postnatal midbrain astrocytes. In addition to this function, Nurr1 is also expressed in adult neurons and is regulated by different types of brain insults, including ischaemia (Neumann-Haefelin et al., 1994; Lin et al., 1996; Law et al., 1992; Johanson et al., 2000; Honkaniemi et al., 1996) and seizures (Crispino et al., 1998).

We have also shown previously that Wnt factors are useful in enhancing induction of neuronal phenotype of cells expressing *Nurr1*. In particular, we found that Wnt-1 is the most efficient Wnt at promoting the proliferation and maturation of dopaminergic precursor and/or stem cells, that Wnt-3a promotes proliferation and/or self-renewal of dopaminergic precursor and/or stem cells and that Wnt-5a is the most efficient at inducing a dopaminergic phenotype in neural stem, precursor or progenitor cells, and in enhancing dopaminergic induction or differentiation in a neuronal cell. See for example WO2004/029229.

Also see Castelo-Branco et al. (2003) PNAS,100:12747-52; Wallén et al. (2003) Genes and Development, 17:3036-47; Timmer et al. (2004) Neurobiology of Disease, 17(2) :163-70; Castelo-Branco et al. (2004) Journal of Cell Science, 117:5731-7; Schulte et al. (2005) Journal of Neurochemistry, 92: 1550-3; Bergman et al. (2005) J Neurochem., 93(5):1132-40; Shariatmadari et al. (2005) Molecular and Cellular Neuroscience, 30(3):437-51; Castelo-Branco (2006) Molecular and Cellular Neuroscience, 31:251-262; Prakash et al. (2006) Development, 133(1):89-98; Rawal et al. (2006) Experimental Cell Research, in press*.*

We previously suggested that factors produced by glial cells in the VM are required for induction, differentiation and maintenance of region-specific neuronal phenotypes (Hall et *al*., 2003).

We show herein that Nurr1 is involved, at a molecular level, in the transition of multipotent stem cells to committed neuronal precursors and in promoting neuronal survival. We have found that Nurr1 promotes cell survival by regulating cell-intrinsic and secreted survival signals, and have identified a number of genes that are upregulated (see e.g. Table 1) and repressed/down-regulated (see e.g. Table 2) by Nurr1. Nurr1 was observed to upregulate genes involved in providing regional identity and in promoting neuronal differentiation. Conversely, Nurr1 downregulates genes involved in proliferation, maintenance of the stem cell state and differentiation into other cell types. Nurr1 was also found to upregulate genes involved in survival and to repress genes involved in cell death, resulting in increased survival and resistance of Nurr1-expressing cells to oxidative stress. Nurr1, and hence Nurr1 upregulated genes, may be at least partly responsible for conferring on cells the capacity to respond to signals from glial cells that promote survival and region-specific differentiation.

The modulation of genes and/or proteins downstream of Nurr1 can affect cellular processes in which Nurr1 is involved, including survival, maturation and/or differentiation of cells.

Further, we show herein that some chemokines increase neural cell proliferation, differentiation and/or survival.

Our results pave the way for the large scale production of midbrain DA neurones *in vitro* and for the future implementation of stem cell replacement strategies in the treatment of Parkinson's disease (Bjorklund and Lindvall 2000; Price and Williams 2001; Arenas, 2002 Rossi and Cattaneo, 2002; Gottlieb et al., 2002), and also treatment of Parkinson's and similar diseases by administration of factors to promote neural cell proliferation, survival, differentiation and/or maturation, thus allowing for the generation of DA neurones *in vivo.*

In WO2006/024947 we have described methods and materials relating to enhanced production of dopamine neurons, by regulating GSK-3β inhibition within the cell other than by means of a ligand for a Frizzled receptor.

PCT/IB2005/003996 describes materials and methods related to dickkopfs and neurogenesis, particularly a method of inducing or promoting dopaminergic neuronal development by enhancing proliferation, self-renewal, dopaminergic neurotransmission, development, induction, survival, differentiation and/or maturation in a neural stem, embryonic stem, progenitor or precursor cell, by modulating the level and/or activity of a Dkk ligand/receptor polypeptide in said cell.

The present invention provides methods of treating stem cells and neural cells, including promoting survival, proliferation, differentiation and/or maturation of stem and neural cells. The methods may be performed *ex vivo or in vitro* (e.g. in culture), for example to generate cells for treatment of neurodegenerative disease or neuronal loss in an individual. Treated cells may then be implanted into the brain of the individual. Methods of treating stem or neural cells may be performed *in vivo*, to treat cells *in situ* in the brain of the individual. The invention also provides materials (substances or compositions) for use in such methods, and methods of screening for such materials.

As used herein, the term "neural cell" means a cell of the nervous system. Normally a neural cell is a neural stem cell, a neural precursor cell, a neural progenitor cell, or a neurone. The terms "neurone" and "neuronal cell" are used interchangeably.

The invention relates especially to dopaminergic neurones, and to cells that can develop into dopaminergic neurones such as adult or embryonic stem cells, neural stem cells, precursor and progenitor cells, especially those expressing combinations of markers characteristic of the VM, including Msx1, lmx1a, lmxlb, Foxa2, Raldh1 or Aldehyde dehydrogenase type 2, Otx2, Pax2, wnt1, wnt-5a, dkk2, sox2, ngn2, RC2, engrailed1, nurr1, pitx3.

Methods of the invention relate especially to treating neural cells and stem cells which can be used and treated in some embodiments of the invention. Stem cells can be treated by methods of the invention in *vitro*, *ex vivo* or *in vivo.*

By "stem cell" is meant any cell type that can self renew and, if it is an ES cell, can give rise to all cells in an individual, or, if it is a multipotent or neural stem cell, can give rise to all cell types in the nervous system, including neurons, astrocytes and oligodendrocytes. A stem cell may express one or more of the following markers: Oct-4; Nanog, Pou5f1 (Chew et al. Molecular & Cellular Biology 2005. 25(14):6031-6046), Sox1-3; stage specific embryonic antigens (SSEA-1, -3, and -4), and the tumor rejection antigens TRA-1-60 and -1-81, as described (Tropepe et al., 2001; Xu et al., 2001). A neural stem cell may express one or more of the following markers: Nestin; the p75 neurotrophin receptor; Notch1, SSEA-1 (Capela and Temple, 2002).

By "neural progenitor cell" is meant a daughter or descendant of a neural stem cell, with a more differentiated phenotype and/or a more reduced differentiation potential compared to the stem cell. By precursor cell it is meant any other cell being or not in a direct lineage relation with neurons during development but that under defined environmental conditions can be induced to transdifferentiate or redifferentiate or acquire a neuronal phenotype. In preferred embodiments, the stem, neural stem, progenitor or precursor cell does not express or express efficiently tyrosine hydroxylase either spontaneously or upon deprivation of mitogens (e.g. bFGF, EGF or serum).

In preferred embodiments of the present invention, the stem or neural cell may be in co-culture with Type 1 astrocytes/glial cells, or in contact with such cells or factors derived from them *in vitro* or *in vivo.* The factor or factors may be supplied by or derived from an immortalized astrocyte/glial cell. The factor or factors may be supplied by or derived from a glial cell line, e.g. an astrocyte or radial glia or immature glial mesencephalic cell line. Cell lines provide a homogenous cell population.

A stem cell, neural stem cell or neural progenitor or precursor cell may be obtained or derived from any embryonic, foetal or adult tissue, including bone marrow, skin, eye, nasal epithelia, or umbilical cord, or region of the nervous system, e.g. from the cerebellum, the ventricular zone, the sub-ventricular zone, the striatum, the midbrain, the hindbrain, the cerebral cortex or the hippocampus. It may be obtained or derived from a vertebrate organism, e.g. from a mammal, which may be human or non-human, such as rabbit, guinea pig, rat, mouse or other rodent, cat, dog, pig, sheep, goat, cattle, horse, or primate; or from an amphibian, such as a frog or salamander; or bird, such as a chicken.

In preferred embodiments of the present invention, adult stem/progenitor/precursor cells are used, *in vitro*, *ex vivo* or in *vivo.* This requires a consenting adult (e.g. from which the cells are obtained) and approval by the appropriate ethical committee. If a human embryo/foetus is used as a source, the human embryo is one that would otherwise be destroyed without use, or stored indefinitely, especially a human embryo created for the purpose of IVF treatment for a couple having difficulty conceiving. IVF generally involves creation of human embryos in a number greater than the number used for implantation and ultimately pregnancy. Such spare embryos may commonly be destroyed. With appropriate consent from the people concerned, in particular the relevant egg donor and/or sperm donor, an embryo that would otherwise be destroyed can be used in an ethically positive way to the benefit of sufferers of severe neurodegenerative disorders such as Parkinson's disease. The present invention itself does not concern the use of a human embryo in any stage of its development. As noted, the present invention minimizes the possible need to employ a material derived directly from a human embryo, whilst allowing for development of valuable therapies for terrible diseases.

In some preferred embodiments, a stem or progenitor or precursor cell treated and/or used in accordance with any aspect of the present invention is obtained from a consenting adult or child for which appropriate consent is given, e.g. a patient with a disorder that is subsequently treated by transplantation back into the patient of neurons generated and/or treated in accordance with the invention to promote endogenous DA neurone development or function.

Cell preparations rich in DA neurones, which may be obtained through methods of the invention, may be used for cell replacement therapy in Parkinson's disease or other disorders, and for studying signalling events in DA neurons and the effects of drugs on DA neurons *in vitro*, for instance in high throughput screening.

The present invention in various aspects provides methods of treating stem or neural cells to modulate Nurr1-regulated genes and/or their expressed proteins.

The invention provides a method of promoting survival, maturation and/or differentiation of a stem or neural cell, comprising
(a) modulating, e.g. increasing activity of a protein encoded by a Nurr1 up-regulated gene (e.g. a protein listed in Table 1) in the cell and/or
(b) modulating, e.g. decreasing activity of a Nurr1 down-regulated gene (e.g. a protein listed in Table 2) in the cell.

In a preferred embodiment the protein whose activity is increased is selected from one or more proteins listed in Table 3. Preferably, the method comprises increasing RDC1 activity. In another preferred embodiment the method comprises increasing activity of Engrailed 1 and/or Runx2.

The activity of one or more than one of the proteins listed in Tables 1 or 3 may be increased, optionally in combination with reducing the activity of one or more than one of the proteins listed in Table 2. Thus, either or both of steps (a) or (b) in the above method may be present. Steps (a) and (b) may be carried out together or sequentially, either (a) then (b) or (b) then (a).

Methods of modulating protein activity are known to the skilled person and any suitable method may be used in the invention.

Protein activity can be increased by increasing expression of the native genomic gene encoding that protein. Protein activity can be increased by expressing the native genomic gene encoding that protein at above basal levels. This may be done by inhibiting or preventing degradation of expressed mRNA or encoded protein or by increasing transcription and/or translation of the gene, and/or by introducing heterologous regulatory sequences into or adjacent to the native regulatory region of the gene, and/or by replacing the native regulatory region of the gene with such heterologous regulatory sequences, e.g. by homologous recombination, and/or by disrupting or downregulating molecules that negatively regulate, block or downregulate transcription, translation or the function of the gene.

Transcription may be increased by providing the cell with increased levels of a transcriptional activator, e.g. by contacting the cell with such an activator or by transformation of the cell with nucleic acid encoding the activator. Alternatively, transcription may be increased by transforming the cell with antisense nucleic acid to a transcriptional inhibitor of the gene.

Protein activity can also be increased by targeting the protein itself, e.g. reducing degradation of the protein, contacting the cell with an activator of the protein or increasing the activity of an endogenous activator, and/or inhibiting the activity of an inhibitor of the protein.

Protein activity can also be increased by introducing nucleic acid encoding the protein into the cell and causing or allowing the nucleic acid to be expressed in the cell. The introduced nucleic acid may be DNA or RNA (e.g. mRNA or cDNA).

Methods of introducing nucleic acid into cells are well known to those skilled in the art.

The introduction of nucleic acid, whether that nucleic acid is linear, branched or circular, may be generally referred to without limitation as "transformation". It may employ any available technique. Suitable techniques may include calcium phosphate transfection, DEAE-Dextran, PEI, electroporation, mechanical techniques such as microinjection, direct DNA uptake, receptor mediated DNA transfer, transduction using retrovirus or other virus and liposome- or lipid-mediated transfection. When introducing a chosen gene construct into a cell, certain considerations must be taken into account, well known to those skilled in the art.

Receptor-mediated gene transfer, in which the nucleic acid is linked to a protein ligand via polylysine, with the ligand being specific for a receptor present on the surface of the target cells, is an example of a technique for specifically targeting nucleic acid to particular cells.

Liposomes can encapsulate RNA, DNA and virions for delivery to cells. Depending on factors such as pH, ionic strength and divalent cations being present, the composition of liposomes may be tailored for targeting of particular cells or tissues. Liposomes include phospholipids and may include lipids and steroids and the composition of each such component may be altered. Targeting of liposomes may also be achieved using a specific binding pair member such as an antibody or binding fragment thereof, a sugar or a glycolipid.

Transformation may be carried out in *vitro*, *in vivo* or *ex vivo*.

Vectors may be used to introduce the nucleic acid into the cell. Suitable vectors can be chosen or constructed, containing appropriate regulatory sequences, including promoter sequences, terminator fragments, polyadenylation sequences, enhancer sequences. Regulatory sequences may drive expression of the gene within the cell.

The gene may be operably-linked to a promotor which drives its expression above basal levels in stem cells, or neural stem, precursor or progenitor cells, or neuronal cells. "Operably linked" means joined as part of the same nucleic acid molecule, suitably positioned and oriented for transcription to be initiated from the promoter. DNA operably linked to a promoter is "under transcriptional initiation regulation" of the promoter.

The introduced nucleic acid may be contained on an extra-genomic vector or it may be incorporated, preferably stably, into the genome. Integration of encoding nucleic acid into the genome may be promoted by including in the transformed nucleic acid sequences which promote recombination with the genome, in accordance with standard techniques. The integrated nucleic acid may include regulatory sequences able to drive expression of the gene in the cell.

A variety of vectors, both viral vectors and plasmid vectors, are known in the art, see e.g. US Patent No. 5,252,479 and WO 93/07282. In particular, a number of viruses have been used as gene transfer vectors, including adenovirus, adeno-associated virus, baculovirus, lentivirus, papovaviruses, such as SV40, vaccinia virus, herpesviruses, including HSV and EBV, and retroviruses, including gibbon ape leukaemia virus, Rous Sarcoma Virus, Venezualian equine enchephalitis virus, Moloney murine leukaemia virus and murine mammary tumourvirus. Many gene therapy protocols in the prior art have used disabled murine retroviruses.

Disabled virus vectors are produced in helper cell lines in which genes required for production of infectious viral particles are expressed. Helper cell lines are generally missing a sequence which is recognised by the mechanism which packages the viral genome and produce virions which contain no nucleic acid. A viral vector which contains an intact packaging signal along with the gene or other sequence to be delivered is packaged in the helper cells into infectious virion particles, which may then be used for the gene delivery.

The vector may be targeted to the specific cells to be treated, or it may contain regulatory elements which are switched on more or less selectively by the target cells. Targeting is particularly useful where the cells to be treated are *in vivo* or are mixed with other non-target cell types.

The gene may be placed under the control of an externally inducible gene promoter to place it under the control of the user. The term "inducible" as applied to a promoter is well understood by those skilled in the art. In essence, expression under the control of an inducible promoter is "switched on" or increased in response to an applied stimulus. The nature of the stimulus varies between promoters. Some inducible promoters cause little or undetectable levels of expression (or no expression) in the absence of the appropriate stimulus. Whatever the level of expression is in the absence of the stimulus, expression from any inducible promoter is increased in the presence of the correct stimulus. An example of an inducible promoter is the Tetracyclin ON/OFF system (Gossen, et al., 1995) in which gene expression is regulated by tetracyclin analogs.

For further details see, for example, Sambrook and Russell, 2001. Many known techniques and protocols for manipulation of nucleic acid, for example in preparation of nucleic acid constructs, mutagenesis, sequencing, introduction of DNA into cells and gene expression, and analysis of proteins, are described in detail Ausubel et al., 1992 or later edition.

Marker genes such as antibiotic resistance or sensitivity genes may be used in identifying clones containing nucleic acid of interest, as is well known in the art. Clones may also be identified or further investigated by binding studies, e.g. by Southern blot hybridisation.

Another means of increasing protein activity is introducing one or more extra copies of the protein into the cell, for example by microinjection or other carrier-based or protein delivery system including cell penetrating peptides, e.g. TAT, transportan, Antennapedia penetratin peptides (Lindsay 2002).

Protein activity can be decreased by inhibiting transcription or translation of the encoding gene. Antisense and RNA interference (RNAi) are well-known techniques. Protein activity can also be decreased by inhibiting the protein itself e.g. using small molecule inhibitors, by increasing the activity of cellular inhibitors of the protein, and/or by increasing degradation of the protein.

Anti-sense oligonucleotides are complementary nucleic acid sequences designed to hybridise to nucleic acid of interest, e.g. pre-mRNA or mature mRNA, thus interfering with the production of polypeptide encoded by a given DNA sequence (e.g. either native polypeptide or a mutant form thereof), so that its expression is reduced or prevented altogether.
Anti-sense techniques may be used to target a coding sequence, a control sequence of a gene, e.g. in the 5' flanking sequence, whereby the antisense oligonucleotides can interfere with control sequences. Anti-sense oligonucleotides may be DNA or RNA and may be of around 14-23 nucleotides, particularly around 15-18 nucleotides, in length. The construction of antisense sequences and their use is described in Peyman and Ulman, 1990; and Crooke, 1992.

Anti-sense nucleic acid molecules can be introduced and targeted to cells using techniques described herein. Other approaches to specific down-regulation of genes are well known, including the use of ribozymes designed to cleave specific nucleic acid sequences. Ribozymes are nuceic acid molecules, actually RNA, which specifically cleave single-stranded RNA, such as mRNA, at defined sequences, and their specificity can be engineered. Hammerhead ribozymes may be preferred because they recognise base sequences of about 11-18 bases in length, and so have greater specificity than ribozymes of the Tetrahymena type which recognise sequences of about 4 bases in length, though the latter type of ribozymes are useful in certain circumstances. References on the use of ribozymes include Marschall, et al. 1994; Hasselhoff, 1988 and Cech, 1988.

An alternative to anti-sense is to use a copy of all or part of the target gene inserted in sense, that is the same, orientation as the target gene, to achieve reduction in expression of the target gene by co-suppression; Angell & Baulcombe 1997; and Voinnet & Baulcombe 1997. Double stranded RNA (dsRNA) has been found to be even more effective in gene silencing than both sense or antisense strands alone (Fire et al 1998). dsRNA mediated silencing is gene specific and is often termed RNA interference (RNAi).

RNA interference is a two step process. First, dsRNA is cleaved within the cell to yield short interfering RNAs (siRNAs) of about 21-23nt length with 5' terminal phosphate and 3' short overhangs (-2nt) The siRNAs target the corresponding mRNA sequence specifically for destruction (Zamore 2001).

RNAi may be also be efficiently induced using chemically synthesized siRNA duplexes of the same structure with 3'-overhang ends (Zamore et al 2000). Synthetic siRNA duplexes have been shown to specifically suppress expression of endogenous and heterologeous genes in a wide range of mammalian cell lines (Elbashir et al. 2001). See also Fire 1999; Sharp 2001; Hammond et al. 2001; and Tuschl 2001.

Methods of producing nucleic acid and protein for use in any method of the invention are described below. See also Sambrook and Russell 2001, for further examples.

A convenient way of producing a polypeptide for use in a method according to the present invention is to express nucleic acid encoding it, by use of the nucleic acid in an expression system.

This may conveniently be achieved by growing a host cell in culture, containing an expression vector, under appropriate conditions which cause or allow expression of the polypeptide. An expression vector in this context is a nucleic acid molecule including nucleic acid encoding a polypeptide of interest and appropriate regulatory sequences for expression of the polypeptide. Polypeptides may also be expressed in *in vitro* systems, such as reticulocyte lysate.

Systems for cloning and expression of a polypeptide in a variety of different host cells are well known. Suitable host cells include bacteria, eukaryotic cells such as mammalian and yeast, and baculovirus systems. Mammalian cell lines available in the art for expression of a heterologous polypeptide include Chinese hamster ovary cells, HeLa cells, baby hamster kidney cells, COS cells and many others. A common, preferred bacterial host is E. *coli*. Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. 'phage, or phagemid, as appropriate. For further details see Sambrook and Russell 2001 and Ausubel et al 1992.

Production of the polypeptide for use in the invention involves causing or allowing expression from the encoding nucleic acid, e.g. by culturing host cells (which may include cells actually transformed although more likely the cells will be descendants of the transformed cells) under conditions for expression of the gene, so that the encoded polypeptide is produced. If the polypeptide is expressed coupled to an appropriate signal leader peptide it may be secreted from the cell into the culture medium. Following production by expression, a polypeptide may be isolated and/or purified from the host cell and/or culture medium, as the case may be, and subsequently used as desired, e.g. in the formulation of a composition which may include one or more additional components, such as a pharmaceutical composition which includes one or more pharmaceutically acceptable excipients, vehicles or carriers (e.g. see below).

Instead of or as well as being used for the production of a polypeptide encoded by a transgene, host cells may be used as a nucleic acid factory to replicate the nucleic acid of interest in order to generate large amounts of it. Multiple copies of nucleic acid of interest may be made within a cell when coupled to an amplifiable gene such as dihyrofolate reductase (DHFR), as is well known. Host cells transformed with nucleic acid of interest, or which are descended from host cells into which nucleic acid was introduced, may be cultured under suitable conditions, e.g. in a fermentor, taken from the culture and subjected to processing to purify the nucleic acid. Following purification, the nucleic acid or one or more fragments thereof may be used as desired.

Methods of the invention may comprise using a modulators to increase or decrease protein activity. As used herein, the term "modulator" means a substance that modulates (increases or decreases) activity of a protein encoded by a Nurr1-regulated (up- or down-regulated gene), by any means including modulation of expression of the gene encoding the protein, or modulation at the protein level. Preferably, a modulator in the present invention increases activity of a protein encoded by a Nurr1-upregulated gene or decreases activity of a protein encoded by a Nurr1-downregulated gene.

The method may comprise contacting the cell to be treated with a modulator, wherein the modulator
(a) alters, e.g. increases activity of a one or more proteins listed in Table 1, and/or
(b) alters, e.g. decreases activity of one or more proteins listed in Table 2.

A "modulator" in the present invention does not consist of Nurr1 protein or nucleic acid, nor of a substance that increases activity of Nurr1 in a cell, nor of a Wnt or Dkk polypeptide or Wnt or Dkk nucleic acid. The methods of increasing and/or decreasing protein activity according to the invention do not require Nurr1 itself to be modulated. The invention allows Nurr1 to be 'by-passed', by regulating a protein downstream of Nurr1. Methods of the invention do not consist of the following: expressing Nurr1 at above basal levels in the neural cell or increasing Nurr1 activity in the cell by any other means, and/or contacting the cell with one or more factors obtainable from a Type 1 astrocyte of the VM, and/or contacting the cell with a Wnt polypeptide. However, use of Nurr1 protein or nucleic acid, or a substance that increases Nurr1 activity in a cell, may be used to treat a neural cell in addition to a modulator, and/or in addition to any other substance or means of treating a neural cell in a method of the invention. Methods of increasing protein activity are described herein and any such suitable method may be used to increase Nurr1 activity. Wnt ligands and factors from Type 1 astrocytes can also be used in methods of the invention, but the invention does not require them and does not consist solely of their use.

The present invention also provides methods of screening for and identifying a modulator of a Nurr1-regulated gene product. The methods of the invention include screening for a substance that modulates protein activity by targeting the protein itself, or a substance that targets the gene that encodes the protein. Methods by which protein activity can be modulated at the protein or nucleic acid level are described elsewhere herein and are known to the person skilled in the art, and a modulator may work by any such method.

Preferably, a screening method of the invention screens for a substance that increases activity of a protein encoded by a Nurr1-upregulated gene, or a substance that decreases activity of a protein encoded by a Nurr1-downregulated gene. This can involve screening for a substance that decreases or increases expression of the Nurr1-regulated gene, accordingly. Nurr1-upregulated and Nurr1-downregulated genes are shown in Tables 1 and 2 respectively. Preferably, the protein encoded by the Nurr1-regulated gene is selected from Table 3, e.g. RDC1, Engrailed-1, Runx2. In the most preferred embodiment, the method screens for a substance able to increase activity of RDC1 protein, e.g. able to increase expression of the RDC1 gene.

One aspect of the invention provides a method of screening for a substance able to modulate expression of a Nurr1-regulated gene, comprising:
(a) contacting DNA containing the promoter of said gene with a test substance, wherein the promoter is operably linked to a gene
(b) determining the level of gene expression from the promoter, and
(c) comparing the level of gene expression in the presence of the test substance with the level of gene expression in the absence of the test substance in comparable conditions,
wherein a difference in the level of gene expression indicates that the test substance is able to modulate expression of the gene.

The method may further comprise identifying the test substance as a modulator of expression of a Nurr1-regulated gene, i.e. as a modulator.

An increase in expression of the gene compared with expression of another gene linked to a different promoter indicates specificity of the substance for modulation of the promoter.

The method may comprise contacting an expression system, such as a host cell containing the gene promoter operably linked to a gene with the test substance, and determining expression of the gene. The gene may be the Nurr1 regulated gene itself or it may be a heterologous gene, e.g. a reporter gene. A "reporter gene" is a gene whose encoded product may be assayed following expression, i.e. a gene which "reports" on promoter activity.

By "promoter" is meant a sequence of nucleotides from which transcription may be initiated of DNA operably linked downstream (i.e. in the 3' direction on the sense strand of double-stranded DNA). The promoter of a Nurr1-regulated gene may comprise one or more fragments of the sequence under the accession number, sufficient to promote gene expression. The promoter of a gene may comprise or consist essentially of a sequence of nucleotides 5' to the gene in the human chromosome, or an equivalent sequence in another species, such as a rat or mouse.

The level of promoter activity is quantifiable for instance by assessment of the amount of mRNA produced by transcription from the promoter or by assessment of the amount of protein product produced by translation of mRNA produced by transcription from the promoter. The amount of a specific mRNA present in an expression system may be determined for example using specific oligonucleotides which are able to hybridise with the mRNA and which are labelled or may be used in a specific amplification reaction such as the polymerase chain reaction (PCR).

PCR comprises steps of denaturation of template nucleic acid (if double-stranded), annealing of primer to target, and polymerisation. The nucleic acid probed or used as template in the amplification reaction may be genomic DNA, cDNA or RNA. Other specific nucleic acid amplification techniques include strand displacement activation, the QB replicase system, the repair chain reaction, the ligase chain reaction and ligation activated transcription. For convenience, and because it is generally preferred, the term PCR is used herein in contexts where other nucleic acid amplification techniques may be applied by those skilled in the art.
Unless the context requires otherwise, reference to PCR should be taken to cover use of any suitable nucleic amplification reaction available in the art.

Use of a reporter gene facilitates determination of promoter activity by reference to protein production. The reporter gene preferably encodes an enzyme which catalyses a reaction that produces a detectable signal, preferably a visually detectable signal, such as a coloured product. Many examples are known, including β-galactosidase and luciferase. β-galactosidase activity may be assayed by production of blue colour on substrate, the assay being by eye or by use of a spectrophotometer to measure absorbance. Fluorescence, for example that produced as a result of luciferase activity or green or red fluorescent proteins, may be quantified using a spectrophotometer or fluorescent microscopy. Radioactive assays may be used, for instance using chloramphenicol acetyltransferase, which may also be used in non-radioactive assays. The presence and/or amount of gene product resulting from expression from the reporter gene may be determined using a molecule able to bind the product, such as an antibody or fragment thereof. The binding molecule may be labelled directly or indirectly using any standard technique.

A promoter construct may be introduced into a cell line using any suitable technique to produce a stable cell line containing the reporter construct integrated into the genome. The cells may be grown and incubated with test compounds for varying times. The cells may be grown in 96 well plates to facilitate the analysis of large numbers of compounds. The cells may then be washed and the reporter gene expression analysed. For some reporters, such as luciferase the cells will be lysed then analysed.

Those skilled in the art are aware of a multitude of possible reporter genes and assay techniques which may be used to determine gene activity. For more examples, see Sambrook and Russell 2001.

Another aspect of the invention provides a method of screening for a substance able to modulate activity of a protein encoded by a Nurr1 regulated gene, including:
(a) bringing into contact the protein and a putative binding molecule or other test substance; and
(b) determining the protein activity, and
(c) comparing the protein activity in the presence of the test substance with the protein activity in the absence of the test substance in comparable reaction medium and conditions,
wherein a difference in protein activity indicates that the test substance is able to modulate the protein activity.

The screening method may further comprise identifying the test substance as a modulator of the protein activity, i.e. as a modulator.

The method of determining protein activity is selected according to the protein used in the screening method.

Observation can be mad of differentiation of a stem, progenitor or neuronal precursor cell that expresses sox2, Lmx1a, Lmx1b, msx1, otx2, pax2, fox2a, Raldh1 or Aldehyde dehydrogenase type 2, wnt1, wnt-5a, dkk2, RC2, nestin, neurogenin2, neurogenin1, mash1, engrailed 1 or nurr1 into dopaminergic neurons by: the acquistion of marker expression such as pitx3, c-ret, tyrosine hydroxylase, dopamine transporter or GIRK channel; morphological differentiation, as assessed by the extension of neurites (expressing MAP2) or axons (expressing neurofilament); increased survival of cells as measured by increased numbers of cells expressing any of the above mentioned markers; increased neurotransmitter (dopamine)release, as measured by HPLC; increased of electrophysiological properties characteristic of mature neurons; increased signalling as measured by increased tyrosine, serine or threonin phosphorylation as measured in protein gels or MALDI-TOF; changes in the cells as detected by Biacore; and/or increased transcription as measured by luciferase or GFP or other reporter systems for instance TOPPFLASH assay to report beta catenin transcription, or promoters involved in dopaminergic differentiation coupled to luciferase or GFP or other reporter systems, such as the genes expressed in progenitor cells.

Assays may be developed to detect the regulation of expression of the chemokines by for instance small molecule libraries. This may be done with the chemokine promoter-reporter systems.

In a screening method according to the invention, the amount of test substance or compound which may be added to an assay of the invention will normally be determined by trial and error depending upon the type of compound used. Typically, from about 0.001 nM to 1mM or more concentrations of putative inhibitor compound may be used, for example from 0.01 nM to 100 µM, e.g. 0.1 to 50 µM, such as about 10 µM. Greater concentrations may be used when a peptide is the test substance. Even a molecule which has a weak effect may be a useful lead compound for further investigation and development. For chemokines a preferred concentration may be up to 2 µg/ml.

A screening method may include purifying and/or isolating a test compound and/or substance of interest from a mixture or extract, i.e. reducing the content of at least one component of the mixture or extract, e.g. a component with which the test substance is naturally associated. The screening or assay method may include determining the ability of one or more fractions of a test mixture or extract to modulate the activity of a protein encoded by a Nurr1-regulated gene.

The purifying and/or isolation may employ any method known to those skilled in the art.

The precise format of any of the screening or assay methods of the present invention may be varied by those of skill in the art using routine skill and knowledge. The skilled person is aware of the need to employ appropriate control experiments.

Compounds which may be screened may be natural or synthetic chemical compounds used in drug screening programmes. Extracts of plants, microbes or other organisms, which contain several characterised or uncharacterised components may also be used.

Combinatorial library technology provides an efficient way of testing a potentially vast number of different substances for ability to modulate an interaction. Such libraries and their use are known in the art, for all manner of natural products, small molecules and peptides, among other. The use of peptide libraries may be preferred in certain circumstances.

An assay may detect the regulation of expression of chemokines by for instance small molecule libraries. This may be done with the chemokine promoter-reporter systems expression in stem, progenitor, precursor cells + exposure to small molecules + read reporter by light emission or fluorescence or immunohistochemistry.

A screening method of the invention may comprise identifying a test substance as a modulator. Following identification of a test substance as a modulator, the substance may be purified and/or investigated further and/or manufactured.
The substance may be developed to obtain peptidyl or non-peptidyl mimetics, e.g. by methods well known to those skilled in the art and discussed herein. Such mimetics are also modulators according to the invention and are provided by the invention.

The designing of mimetics to a known pharmaceutically active compound is a known approach to the development of pharmaceuticals based on a "lead" compound. This might be desirable where the active compound is difficult or expensive to synthesise or where it is unsuitable for a particular method of administration.

There are several steps commonly taken in the design of a mimetic from a compound having a given target property. Firstly, particular parts of the compound that are critical and/or important in determining the target property are determined. In the case of a peptide, this can be done by systematically varying amino acid residues in the peptide, e.g. by substituting each residue in turn (for example with alanine i.e. 'alanine scanning'). These parts or residues constituting the active region of the compound are known as its "pharmacophore".

Once the pharmacophore has been found, its structure is modelled according to its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can be used in this modelling process.

In a variant of the above approach, the three-dimensional structure of a ligand and its binding partner (the Nurr1-regulated protein) are modelled. This can be especially useful where the ligand and/or binding partner change conformation on binding, allowing the model to take account of this the design of the mimetic.

A mimetic or modulator may, as a further step, be formulated into a medicament and optionally used in a method of treatment as described herein.

The invention provides a modulator identified by a screening method above, for use in a method of treating a neural cell and/or a method of treatment of a neurodegenerative disease or disorder. A modulator may be provided in an isolated and/or purified form, i.e. substantially pure. This may include being in a composition where it represents at least about 90% active ingredient, more preferably at least about 95%, more preferably at least about 98%. Such a composition is preferably a pharmaceutical composition and may include inert carrier materials or other pharmaceutically and physiologically acceptable excipients. An active ingredient means a pharmaceutically active substance; as opposed to e.g. a buffer or carrier material included to stabilise the active ingredient or facilitate its administration.

Thus, the present invention extends in various aspects not only to a substance identified as a modulator in accordance with what is disclosed herein, but also a pharmaceutical composition, medicament, drug or other composition comprising such a substance, a method comprising administration of such a composition to a patient, for instance in treatment (which may include preventative treatment) of neurodegenerative disease or neuronal loss, use of such a substance in manufacture of a composition for administration to such a patient, and a method of making a pharmaceutical composition comprising admixing such a substance with a pharmaceutically acceptable excipient, vehicle or carrier, and optionally other ingredients. Pharmaceutical compositions and therapeutic methods are part of the present invention and are described in more detail below.

Further aspects of the invention relate to contacting a stem or neural cell with one or more chemokines to promote cell proliferation, differentiation, maturation and/or survival. Chemokines that can be used in methods according to the invention include BLC-1, SDF1, IL-8, an α-chemokine, optionally an α-chemokine selected from the group consisting of CXCL6, CXCL8, CXCL11, CXCL12 and CXCL13, a β-chemokine, optionally a β-chemokine selected from the group consisting of CCL2 and CCL7. Preferred chemokines are ligands for CCR2, RDC1, CXCR2, CXCR3, CXCR4 and/or CXCR5.

The invention provides a method of promoting cell proliferation and increasing the number of TH-expressing cells in a group of stem or neural cells, comprising contacting the cells with one or more chemokines selected from the group consisting of BLC-1, SDF1, IL-8, an α-chemokine, e.g. one or more of those disclosed herein for use in the invention, and/or a α-chemokine, e.g. one or more of those disclosed herein for use in the invention.

Preferably, the neural cells are ventral midbrain (ventral mesencephalic) cells. The group of cells may be a mixed primary culture containing neural cells at various levels of differentiation, or a culture primarily containing embryonic or adult stem cells, neural stem cells or precursor or progenitor cells. Methods of the invention can be *in vitro, ex vivo* or *in vivo.* The cells may be in *situ* in the brain of an individual.

A chemokine as disclosed herein may increase TH+ cell number by promoting survival of neural cells, including DA neurones. TH is the rate limiting enzyme in the synthesis of dopamine and therefore a marker of dopaminergic neurons. One should always use other markers to identify the cells as midbrain dopaminergic neurons. These may be: TH, dopamine transporter, GIRK, pitx3, nurr1, engrailed1, lmxlb, lmxla. Prevention of cell death may allow differentiation programs to continue in the cells.

Additionally, the chemokines may promote differentiation and maturation of embryonic or adult stem cells, neural stem cells, precursor or progenitor cells.

In a preferred embodiment, the invention provides a method of promoting proliferation and differentiation of neural precursor or progenitor cells, comprising contacting the cells with a chemokine or other substance or agent as disclosed herein.

The invention also provides a method of promoting the differentiation or maturation of a stem cell, neural stem cell or dopaminergic precursor or progenitor cell into a neurone, comprising contacting the cell with CXCL12 or other α-chemokine or β-chemokine, optionally those identified herein.

In a preferred embodiment, differentiation or maturation into a dopaminergic neurone is promoted.

Contacting a neurone with CXCL12 or other chemokine also promotes neuritogenesis, and accordingly the invention provides a method of promoting neuritogenesis in a precursor, progenitor or neuronal cell, comprising contacting the cell with CXCL6 or other chemokine. Preferably, the cell is of the dopaminergic lineage.

Thus, CXCL12 or other chemokine can be used to generate a neurite-rich neurone, preferably a dopaminergic neurone.

Contacting a cell with a chemokine can involve contacting the cell with the chemokine protein. The protein itself may be used, or nucleic acid encoding the protein may be introduced into the cell and expressed. Methods of contacting cells with proteins are further described elsewhere herein.

Any method of treating a stem or neural cell according to the invention can be used in combination with any another such method, either together or sequentially.

In methods of the invention involving treating neural cells, an additional or simultaneous step may be carried out, comprising contacting the cell with a Wnt polypeptide.

The induction of specific neuronal phenotypes involves the integration of both genetic and epigenetic signals. Wnt-5a is part of such signal and members of the Wnt family of proteins, including Wnt-1, -3a and -5a are developmentally regulated and differentially control the development of midbrain DA neurons. In our previous experiments, partially purified Wnt-1 and -5a, but not Wnt-3a, increased the number of E14.5 midbrain DA neurons by two different mechanisms. While Wnt-1 predominantly increased the proliferation of Nurr1 precursors, Wnt-5a mainly increased the proportion of Nurr1 precursors that acquired a neuronal dopaminergic phenotype. In agreement with this, Wnt5a was as efficient as midbrain astrocytes/early glial cells at inducing DA neurons in Nurr1-expressing midbrain or cortical E13.5 precursors. Moreover, the cysteine rich domain of Frizzled 8 efficiently blocked the basal and the VM T1A-, Wnt-1 or Wnt5a-mediated effects on the increase of cells with a dopaminergic phenotype in Nurr1-expressing neural precursor cultures, and the effect of endogenous Wnts on neural stem cells or FGF-8 expanded Nurr1+ midbrain neurospheres. Thus, our data previously provided indication that Wnt-1 and Wnt-5a regulate by two partially different mechanisms the generation of neurons with a dopaminergic phenotype in Nurr1-expressing precursors/stem cells. These findings place Wnt ligands as key regulators of proliferation and self-renewal, differentiation and fate decisions during ventral midbrain neurogenesis.

Means of contacting a cell with a Wnt polypeptide or a chemokine include provision of purified and/or recombinant Wnt polypeptide or chemokine to a culture comprising the neural cell, or to such a cell *in vivo*. Contacting a cell with a Wnt polypeptide or chemokine may comprise introducing one or more copies of Wnt or chemokine nucleic acid into the cell, and allowing the protein to be expressed, or introducing the protein itself into the cell. Methods of transforming cells with nucleic acid and introducing proteins into cells are described herein. Contacting with a Wnt polypeptide or chemokine may be by means of providing in *vivo* or within a culture comprising the neural cell a cell that produces the Wnt polypeptide or chemokine. The cell that produces the Wnt polypeptide or chemokine may be a recombinant host cell that produces Wnt polypeptide or chemokine by recombinant expression. A co-cultured host cell may be transformed with nucleic acid encoding a Wnt polypeptide or chemokine, and/or the co-cultured cell may contain introduced Wnt protein or chemokine. The nucleic acid or protein may be introduced into the cell in accordance with available techniques in the art, examples of which are described herein. The co-cultured or host cell may be another neural cell e.g. a stem, neural stem, progenitor, precursor or neuronal cell. Contact with a Wnt protein or chemokine may also be by means of upregulating its expression in the cell or by downregulating or inhibiting an inhibitor molecule of the Wnt protein or chemokine. Thus contact with a Wnt protein may arise by decreasing expression or activity of Wnt-interacting molecules, such as SFRP, WIF, dkk or Cerberus (Martinez Arias et al., 1999; and the Wnt home page at http://www.stanford.edu/∼rnusse/wntwindow.html or findable using any web browser).

As used herein, a "Wnt polypeptide", "Wnt glycoprotein" or "Wnt ligand" refers to a member of the Wingless-int family of secreted proteins that regulate cell-to-cell interactions. Wnts are highly conserved from Drosophila and Caenorhabditis elegans, to Xenopus, zebra fish and mammals. The 19 Wnt proteins currently known in mammals bind to two cell surface receptor types: the seven transmembrane domain Frizzled receptor family, currently formed by 10 receptors, and the Low density lipoprotein-receptor related proteins (LRP) 5 and 6 and the kremen 1 and 2 receptors. The signal conveyed by Wnts is transduced via three known signaling pathways: (1) the so called canonical signaling pathway, in which GSK3 beta is inhibited, does not phosphorylate beta-catenin, which is then not degraded and is translocated to the nucleus to form a complex with TCF and activate transcription of Wnt target genes. (2) the planar polarity and convergence-extension pathway, via Jnk. (3) and the inositol 1,4,5 triphosphate (IP3)/calcium pathway, in which calcineurin dephosphorylates and activates the nuclear factor of activated T cells (NF-AT)(Saneyoshi et al., 2002). For review see the Wnt home page, findable on the web using any available browser (currently at www.stanford.edu/∼rnusse/wntwindow.html). Other co-receptors involved in Wnt signaling include the tyrosine kinase receptor Ror1 and Ror2 (Oishi I et al., 2003), the derailed/RYK receptor family (Yoshikawa et al., 2003), which encode catalytically inactive receptor tyrosine kinases.

In some preferred embodiments of the various aspects of the present invention the Wnt ligand is a Wnt1 ligand. Human Wnt1 amino acid sequence is available under GenBank reference Swiss protein accession number P04628 and encoding nucleic acid under reference X03072.1 for DNA and NM_005430.2 for RNA.

In some preferred embodiments of the various aspects of the present invention the Wnt ligand is a Wnt5a ligand. Human Wnt5a amino acid sequence is available under GenBank reference Swiss protein accession number P41221. and encoding nucleic acid under reference AI634753.1 AK021503 L20861 L20861.1 U39837.1 for DNA and NM_003392 for RNA.

Although not preferred for generation of DA neurons, some preferred embodiments of the present invention may employ a Wnt3a ligand. Various aspects and embodiments of the invention analogous to those disclosed herein for generation and use of DA neurons are provided by the present invention in which a Wnt3a ligand is used to maintain the proliferation or self-renewal of stem/progenitor cells and/or allow or induce their differentiation into other, i.e. non-dopaminergic, neuronal phenotypes. As demonstrated by the experiments described herein, Wnt3a decreases the number of *Nurr*-*1* expressing progenitors that give rise to DA neurons. However, since the total number of neurons is not decreased other neuronal phenotypes may be produced, e.g. dorsal midbrain phenotypes, including serotonergic neurons. Loss of serotonergic neurons is associated with depression, so neurons generated by methods comprising use of a Wnt3a ligand, and/or a Wnt3a ligand itself, may be used in therapies e.g. of depression.

Human Wnt3a amino acid sequence is available under GenBank reference SwissProt Accession No.P56704 and encoding nucleic acid under reference AB060284 AB060284.1 AK056278 AK056278.1 for DNA and NM_033131 for mRNA.

A wild-type Wnt ligand may be employed, or a variant or derivative, e.g. by addition, deletion, substitution and/or insertion of one or more amino acids, provided the function of enhancing development of a dopaminergic neuronal fate in a stem cell, neural stem cell or neural progenitor or precursor cell is retained.

In methods of the invention, the stem or neural cell may be optionally contacted with one or more factors supplied by or derived from a Type 1 astrocyte/glial cell. The factor or factors may be provided by co-culturing or contacting the stem, progenitor or precursor cell or neuronal cell with a Type 1 astrocyte/glial cell, or by contact with such cells or factors derived from them *in vitro* or *in vivo.* The factor or factors may be supplied by or derived from a glial cell line, e.g. an astrocyte or radial glia or immature glial mesencephalic cell line. Cell lines provide a homogenous cell population.

In methods of the invention, the stem or neural cell may optionally be treated to modulate GSK-3β inhibition within the cell other than by means of a ligand for a Frizzled receptor, e.g. in accordance with disclosure within W02006/024947.

In methods of the invention, the stem or neural cell may optionally be treated to modulate the level and/or activity of a Dkk ligand/receptor polypeptide in said cell, e.g. in accordance with disclosure within PCT/IB2005/003996.

Cells generated in accordance with the present invention may be used in toxicity studies and/or drug discovery, wherein they are treated with a test substance and the effect of the substance on cell viability, survival, proliferation, differentiation or maturation, and/or one or more properties or phenotypes is determined.

Methods of the invention involving treating a stem or neural cell, particularly when *in vitro* or *ex vivo,* may comprise a further step of measuring or detecting survival, proliferation, differentiation and/or maturation of the neural cell.

The further step may include detecting a marker for the neuronal fate. b-tubulin III (TuJ1) is one marker of the neuronal fate (Menezes, et al., 1994). Other neuronal markers include neurofilament and MAP2. If a particular neuronal phenotype is induced, the marker should be specific for that phenotype. For the dopaminergic fate, expression of tyrosine hydroxylase (TH), dopamine transporter (DAT) and dopamine receptors may be detected e.g. by immunoreactivity or in *situ* hybridization. Contents and/or release of dopamine and metabolites may be detected e.g. by High Pressure Liquid Chromatography (HPLC) (Cooper, et al., 1996). The absence of Dopamine β hydroxylase and GABA or GAD (in the presence of TH/dopamine/DAT) is also indicative of dopaminergic fate. Additional markers include Aldehyde dehydrogenase type 2 (ADH-2), Lmx1b and Ptx3.

Detection of a marker may be carried out according to any method known to those skilled in the art. The detection method may employ a specific binding member capable of binding to a nucleic acid sequence encoding the marker, the specific binding member comprising a nucleic acid probe hybridisable with the sequence, or an immunoglobulin/antibody domain with specificity for the nucleic acid sequence or the polypeptide encoded by it, the specific binding member being labelled so that binding of the specific binding member to the sequence or polypeptide is detectable. A "specific binding member" has a particular specificity for the marker and in normal conditions binds to the marker in preference to other species. Alternatively, where the marker is a specific mRNA, it may be detected by binding to specific oligonucleotide primers and amplification in e.g. the polymerase chain reaction.

Nucleic acid probes and primers may hybridize with the marker under stringent conditions. Suitable conditions include, e.g. for detection of marker sequences that are about 80-90% identical, hybridization overnight at 42C in 0.25M Na₂HPO₄, pH 7.2, 6.5% SDS, 10% dextran sulfate and a final wash at 55C in 0.1X SSC, 0.1% SDS. For detection of marker sequences that are greater than about 90% identical, suitable conditions include hybridization overnight at 65C in 0.25M Na₂HPO₄, pH 7.2, 6.5% SDS, 10% dextran sulfate and a final wash at 60C in 0.1X SSC, 0.1% SDS.

Various nucleic acids and polypeptides have been referred to above for use in the methods of the present invention, and accession numbers for the corresponding sequences are provided herein.

Nucleic acid for use in a method of the invention is generally provided as an isolate, in isolated and/or purified form, or free or substantially free of material with which it is naturally associated, such as free or substantially free of nucleic acid flanking the gene in the human genome, except one or more regulatory sequence(s) for expression. Nucleic acid may be wholly or partially synthetic and may include genomic DNA, cDNA or RNA e.g mRNA.

A nucleic acid sequence for use in the invention may be that given under the Accession number, or it may be a mutant, variant, derivative or allele of the sequence shown. The sequence may differ from that shown by a change which is one or more of addition, insertion, deletion and substitution of one or more nucleotides of the sequence shown. Changes to a nucleotide sequence may result in an amino acid change at the protein level, or not, as determined by the genetic code. On the other hand the encoded polypeptide may comprise an amino acid sequence which differs by one or more amino acid residues from the amino acid sequence shown under the Accession number. Such polypeptides are discussed below. Nucleic acid encoding such a polypeptide may show greater than about 60% nucleotide sequence identity with the coding sequence shown, greater than about 70% identity, greater than about 80% identity, greater than about 90% identity or greater than about 95% identity. Sequence identities can be calculated using the BLAST program with default parameters, available on the Internet at http://www.ncbi.nlm.nih.gov/blast/bl2seq/bl2.html, or findable using any web browser.

The present invention extends to nucleic acid that hybridizes with any one or more of the specific sequences disclosed herein under stringent conditions. Suitable conditions include, e.g. for detection of sequences that are about 80-90% identical suitable conditions include hybridization overnight at 42°C in 0.25M Na₂HPO₄, pH 7.2, 6.5% SDS, 10% dextran sulfate and a final wash at 55°C in 0.1X SSC, 0.1% SDS. For detection of sequences that are greater than about 90% identical, suitable conditions include hybridization overnight at 65°C in 0.25M Na₂HPO₄, pH 7.2, 6.5% SDS, 10% dextran sulfate and a final wash at 60°C in 0.1X SSC, 0.1% SDS.

A protein for use in a method described herein is generally in isolated and/or purified form, free or substantially free of material with which it is naturally associated, such as other polypeptides or (for example if produced by expression in a prokaryotic cell) lacking in native glycosylation, e.g. unglycosylated.

The terms "protein" and "polypeptide" are used interchangeably herein, except where context requires otherwise.

Polypeptides which are amino acid sequence variants, alleles, derivatives or mutants of the proteins having accession numbers disclosed herein can also be used in any of the methods described herein. Where the nucleic acid sequence is given under the accession number, the given polypeptide sequence can be found by translation. A polypeptide which is a variant, allele, derivative or mutant may have an amino acid sequence which differs from the sequence deposited under the accession number by one or more of addition, substitution, deletion and insertion of one or more amino acids. Preferred such polypeptides have the function of the sequence given under the accession number, that is to say have one or more of the following properties: immunological cross-reactivity with an antibody reactive to the given polypeptide; sharing an epitope with the given polypeptide (as determined for example by immunological cross-reactivity between the two polypeptides); a biological activity which is inhibited by an antibody raised against the given polypeptide; one or more functions of the given polypeptide. Alteration of sequence may change the nature and/or level of activity and/or stability of the protein.

A polypeptide which is an amino acid sequence variant, allele, derivative or mutant of the given amino acid sequence may comprise an amino acid sequence which shares greater than about 35% sequence identity with the sequence shown, greater than about 40%, greater than about 50%, greater than about 60%, greater than about 70%, greater than about 80%, greater than about 90% or greater than about 95%. The sequence may share greater than about 60% similarity, greater than about 70% similarity, greater than about 80% similarity or greater than about 90% similarity with the amino acid sequence.
Amino acid similarity is generally defined with reference to the algorithm GAP (Genetics Computer Group, Madison, WI) as noted above, or the TBLASTN program, of Altschul et al. 1990. Similarity allows for "conservative variation", i.e. substitution of one hydrophobic residue such as isoleucine, valine, leucine or methionine for another, or the substitution of one polar residue for another, such as arginine for lysine, glutamic for aspartic acid, or glutamine for asparagine. Particular amino acid sequence variants may differ from that given under the accession number shown by insertion, addition, substitution or deletion of 1 amino acid, 2, 3, 4, 5-10, 10-20 20-30, 30-50, 50-100, 100-150, or more than 150 amino acids.

Sequence comparison may be made over the full-length of the relevant sequence shown herein, or may more preferably be over a contiguous sequence of about or greater than about 20, 25, 30, 33, 40, 50, 67, 133, 167, 200, 233, 267, 300, 333, 400, 450, 500, 600, 700, 800, 900, 1000, 1100, 1200, 1300, 1400, 1500, 1600, or more amino acids or nucleotide triplets, compared with the relevant amino acid sequence or nucleotide sequence as the case may be.

Active portions, fragments, derivatives and functional mimetics of the polypeptides shown under the accession numbers can also be used. An "active portion" of a polypeptide means a peptide which is less than said full length polypeptide, but which retains a biological activity, such as a biological activity selected from binding to ligand, involvement in endocytosis. Thus an active portion of a polypeptide may, in one embodiment, include the transmembrane domain and the portion of the cytoplasmic tail involved in endocytosis. Such an active fragment may be included as part of a fusion protein, e.g. including a binding portion for a different ligand. In different embodiments, combinations of LDL and EGF motifs may be included in a molecule to confer on the molecule different binding specificities.

The term "functional mimetic" means a substance which may not contain an active portion of the relevant amino acid sequence, and probably is not a peptide at all, but which retains in qualitative terms biological activity of natural polypeptide. The design and screening of candidate mimetics is described in detail herein.

A "fragment" of a polypeptide generally means a stretch of amino acid residues of at least about five contiguous amino acids, often at least about seven contiguous amino acids, typically at least about nine contiguous amino acids, more preferably at least about 13 contiguous amino acids, and, more preferably, at least about 20 to 30 or more contiguous amino acids. Fragments of a polypeptide sequence may include antigenic determinants or epitopes useful for raising antibodies to a portion of the amino acid sequence. Alanine scans are commonly used to find and refine peptide motifs within polypeptides, this involving the systematic replacement of each residue in turn with the amino acid alanine, followed by an assessment of biological activity.

A polypeptide for use in a method according to the present invention may be isolated and/or purified (e.g. using an antibody) for instance after production by expression from encoding nucleic acid (for which see below). Thus, a polypeptide may be provided free or substantially free from contaminants with which it is naturally associated (if it is a naturally-occurring polypeptide). A polypeptide may be provided free or substantially free of other polypeptides.

Polypeptides according to the present invention may be generated wholly or partly by chemical synthesis. Standard liquid or, preferably, solid-phase peptide synthesis methods are described, for example, in Stewart and Young, 1984; in Bodanzsky and Bodanzsky, 1984; and Applied Biosystems 430A Users Manual, ABI Inc., Foster City, California. Proteins or peptides can be prepared in solution, by the liquid phase method or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof.

In a further aspect, the present invention provides a neural cell treated in accordance with any one of the methods disclosed herein, or generated by such a method, preferably *in vitro* or *ex vivo*. The invention provides a neural cell generated by a method according to the invention, including a differentiated cell that was treated at an earlier stage of differentiation, or a daughter cell of a cell treated by a method according to the invention. The invention provides a neural cell that is a descendant of a cell that was treated by a method of the invention. The neural cell is preferably isolated from the body, *ex vivo* or *in vitro*, not *in situ* in the brain of an individual.

The neural cell may have a primitive neuronal phenotype. It may be capable of giving rise to a plurality of distinct neuronal phenotypes. The neural cell may have a particular neuronal phenotype, the phenotype being influenced by the particular method of the invention, e.g. in some embodiments, the Wnt ligand and/or the type of astrocytes/glial cells from which the factor or factors which contacted the neural cell were supplied or derived, and/or by the type of astrocyte/glial cell with which the neural cell was co-cultured or contacted. In preferred embodiments, the neural cell has a dopaminergic phenotype, and most preferably the neural cell is a neurone.

The neural cell provided by the invention may contain nucleic acid encoding a chemokine identified herein, a Wnt polypeptide, a protein encoded by a Nurr1-upregulated gene, and/or another molecule with neuroprotective or neuroregenerative properties operably linked to a promoter which is capable of driving expression of the molecule in the neurone. The promoter may be an inducible promoter, e.g. the TetON chimaeric promoter, so that any damaging over-expression may be prevented. The promoter may be associated with a specific neuronal phenotype, e.g. the TH promoter or the *Nurr1* promoter.

The encoded molecule may be such that its expression renders the neurone independent of its environment, i.e. such that its survival is not dependent on the presence of one or more factors or conditions in e.g. the neural environment into which it is to be implanted. By way of example, the neurone may contain nucleic acid encoding one or more of the neuroprotective or neuroregenerative molecules described below operably linked to a promoter which is capable of driving expression of the molecule in the neurone.

In addition or alternatively, expression of the encoded molecule may function in neuroprotection or neuroregeneration of the cellular environment surrounding that neurone. In this way, the neurone may be used in a combined cell and gene therapy approach to deliver molecules with neuroprotective and neuroregenerative properties.

Examples of molecules with neuroprotective and neuroregenerative properties include:
(i) neurotropic factors able to compensate for and prevent neurodegeneration. One example is glial derived neurotropic growth factor (GDNF) which is a potent neural survival factor, promotes sprouting from DA neurons and increases tyrosine hydroxylase expression (Tomac, et al.,1995; Arenas, et al., 1995). By enhancing axonal elongation GDNF, GDNF may increase the ability of the neurons to inervate their local environment. Other neurotropic molecules of the GDNF family include Neurturin, Persephin and Artemin. Neurotropic molecules of the neurotropin family include nerve growth factor (NGF), brain derived neurotropic factor (BDNF), and neurotropin-3, -4/5 and -6. Other factors with neurotrophic activity include members of the FGF family for instance FGF2, 4, 8 and 20; members of the Wnt family, including Wnt-1, -2, -5a, -3a and 7a; members of the BMP family, including BMP2, 4, 5 and 7; and members of the TGFalpha/beta family.
(ii) antiapoptotic molecules. Bcl2 which plays a central role in cell death. Over-expression of Bcl2 protects neurons from naturally occurring cell death and ischemia (Martinou, et al., 1994). Another antiapoptotic molecule is BclX-L.
(iii) axon regenerating and/or elongating and/or guiding molecules which assist the neurone in innervating and forming connections with its environment, e.g. ephrins. Ephrins define a class of membrane-bound ligands capable of activating tyrosine kinase receptors. Ephrins have been implicated in neural development (Irving, et al., 1996; Krull, et al., 1997; Frisen, et al., 1998; Gao, et al., 1996; Torres, et al., 1998; Winslow, et al., 1995; Yue, et al., 1999.
(iv) transcription factors, e.g. the homeobox domain protein Ptx3 (Smidt, et al., 1997), Msx1, Lmx1a, Lmx1b, Pax2, Pax5, Pax8, or engrailed 1 or 2 (Wurst and Bally-Cuif, 2001; Rhinn and Brand, 2001).

A neurone in accordance with or for use in the present invention may be substantially free from one or more other cell types, e.g. from stem, neural stem, precursor or progenitor cells. Neuronal cells may be separated from neural stem or progenitor cells using any technique known to those skilled in the art, including those based on the recognition of extracellular epitopes by antibodies and magnetic beads or fluorescence activated cell sorting (FACS). By way of example, antibodies against extracellular regions of molecules found on stem, neural stem, precursor or progenitor cells but not on neurons may be employed. Such molecules include Notch 1, CD133, SSEA1, prominin1/2 and the glial cell line derived neurotrophic factor receptors GFR alphas or NCAM. Stem cells bound to antibodies may be lysed by exposure to complement, or separated by, e.g. magnetic sorting (Johansson, et al., 1999). If antibodies which are xenogeneic to the intended recipient of the neurons are used, then any e.g. stem, neural stem or progenitor or precursor cells which escape such a cell sorting procedure are labelled with xenogeneic antibodies and are prime targets for the recipient's immune system. Alternatively, cells that acquire the desired phenotype could also be separated by antibodies against extracellular epitopes or by the expression of transgenes including fluorescent proteins under the control of a cell type specific promoter. By way of example DA neurons could be isolated with fluorescent proteins expressed under the control of TH, DAT, Ptx3 or other promoters specifically used by DA neurons.

The invention provides a neural cell treated or generated by a method of the invention, for use in medical treatment. Methods of treatment are detailed below.

The invention also provides a substance for use in medical treatment, wherein the substance is selected from the group consisting of: nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, or nucleic acid encoding such a chemokine. The invention also provides use of such a substance for treating a stem or neural cell in a method of the invention. This cell could either be used for drug discovery/toxicology testing and for cell therapy.

A nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, nucleic acid encoding such a chemokine, and/or a neural cell according to the invention can be manufactured and/or used in preparation i.e. manufacture or formulation, of a composition such as a medicament, pharmaceutical composition or drug.

The present invention provides use of nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, nucleic acid encoding such a chemokine and/or a neural cell treated by a method of the invention, in the manufacture of a medicament for treating neurodegenerative disease or neuronal loss.

The present invention also provides a method of manufacturing a medicament for treating neurodegenerative disease or neuronal loss, comprising:
(a) treating a neural cell using a method according to claim 21; and
(b) formulating the neural cell, or a descendent of the cell, into a composition comprising a pharmacologically acceptable excipient.

Methods of treatment in which neural cells and/or therapeutic substances of the invention can be used are described more fully elsewhere herein.

Thus, in various further aspects, the present invention provides a pharmaceutical composition, medicament, drug or other composition for use in a therapeutic method described herein, the composition comprising nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, nucleic acid encoding such a chemokine, and/or a neural cell identified herein. The invention also provides a method of making a pharmaceutical composition comprising admixing nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, nucleic acid encoding such a chemokine and/or a neural cell with a pharmaceutically acceptable excipient, vehicle or carrier, and optionally other ingredients.

The cells or substances may be used as sole active agents or in combination with one another or with any other active substance. Whatever the substance used in a method of medical treatment of the present invention, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

A substance or composition may be administered alone or in combination with other treatments, either simultaneously or sequentially dependent upon the condition to be treated.

Pharmaceutical compositions according to the present invention, and for use in accordance with the present invention, may include, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous or intravenous. Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.
Liposomes, particularly cationic liposomes, may be used in carrier formulations.

Examples of techniques and protocols mentioned above can be found in Remington's Pharmaceutical Sciences.

Various aspects of the invention provide methods of medical treatment, particularly treatment of neurodegenerative disease or neuronal loss. In a preferred embodiment the neurodegenerative disease is Parkinson's disease or parkinsonian syndrome.

The invention provides a method of treating neurodegenerative disease or neuronal loss in an individual, comprising
(a) treating a neural cell using an *in vitro* or *ex vivo* method according to the invention; and
(b) administering the neural cell, or a descendent of the cell, to the individual.

The invention provides a method of treating neurodegenerative disease or neuronal loss in an individual, comprising administering a neural cell provided by the invention to the individual.

The administration may comprise implanting the neural cell into the brain of the individual. Suitable grafting techniques are known in the art. Implanted cells can replace lost neural tissue and thereby ameliorate neurodegenerative disease or neuronal loss, or prevent or slow the loss of neural tissue or neurodegeneration in an individual.

A neural cell treated by a method of the invention and/or used in a method of treatment in the invention may be endogenous to the individual to be treated, i.e. previously obtained from that individual, or descended from tissue or cells previously obtained from that individual. An endogenous cell has the same genetic material as cells of the individual being treated, thus limiting problems of rejection by the immune system. Alternatively the cell may be exogenous to the individual. Sources of stem cells and neural cells that may be treated or used in a method of the invention are described elsewhere herein.

While transplantation of neural tissue is an attractive technique for replacing lost neuronal tissue in individuals who have neurodegenerative disorders or neuronal loss, an alternative treatment is direct infusion of substances required to promote regeneration, repair or guide the development and/or recruitment of stem or progenitor or precursor cells, or the administration of drugs that regulate those functions.

The present invention provides a method of treating neurodegenerative disease or neuronal loss in an individual, the method comprising administering to the individual one or more substances selected from the group consisting of:
nucleic acid encoding a Nurr1-upregulated gene, a protein encoded by a Nurr1-upregulated gene, double stranded RNA encoding a Nurr-1 downregulated gene, nucleic acid complementary to a sequence encoding a Nurr-1 downregulated gene, a CCL2, CCL7, BLC-1/CXCL13, SDF1/CXCL12, I-Tac/CXCL11, IL-8/CXCL8 or IL-6/CXCL6 chemokine, and nucleic acid encoding such a chemokine. Thus, neural cells *in situ* in an individual may be treated by administration of the substance to the individual. As above, the cell may be endogenous or exogenous to the individual being treated, and may or may not be an implanted or grafted cell.

The ability to promote survival, proliferation, differentiation and/or maturation of stem cells or neural stem, progenitor or precursor cells, or neurones prior to and/or following transplantation, ameliorates the biasing of transplanted stem cells to differentiate into astroglial fates when grafted in the adult brain.

Accordingly, materials and methods of the invention may be used to treat neural cells post-transplantation, i.e. *in situ* in the individual to be treated. Post-implantation treatments may be performed using methods of the invention whether or not the implanted cells were treated by methods of the invention prior to being implanted.

Alternatively or additionally, materials and methods of the invention can be used on non-transplanted cells *in situ* in the brain of an individual to be treated. A substances or composition of the invention may be administered to an individual in a localised manner to the brain or other desired site or may be delivered in a manner in which it reaches the brain or other neural cells. Preferably, the substance or composition is targeted to the neural cells to be treated.

Targeting therapies may be used to deliver the active substance more specifically to certain types of cell, by the use of targeting systems such as antibody or cell specific ligands. Targeting may be desirable for a variety of reasons, for example if the agent is unacceptably toxic, or if it would otherwise require too high a dosage, or if it would not otherwise be able to enter the target cells.

Combination treatments are possible. For example, one or more methods of the invention may be performed to prepare cells for administering into an individual. Following administration, the neural cells may be treated *in situ* according to one or more methods of the invention, to reduce death of the administered cells and promote further proliferation, maturation and/or differentiation.

In situ treatment with CXCL6 is useful for promoting neuritogenesis of DA neurones. This may promote integration of the implanted cells into the brain. CXCL6 can also be used as a treatment on cells before their transplantation into the body, although there may be a risk of damage to the neurites during administration.

Various further aspects and embodiments of the present invention will be apparent to those skilled in the art in view of the present disclosure. All documents mentioned in this specification are incorporated herein by reference in their entirety.

Certain aspects and embodiments of the invention will now be illustrated by way of example and with reference to the figures described below.

### Brief Description of the Figures

Figure 1 shows that Nurr1 regulates the expression of stem/progenitor cell markers and promotes differentiation.
Figure 1A: Immunocytochemical detection of Nestin, tubulin β III (Tuj1), tenascin-C (TN-C), in both control- (pB) and Nurr1- (c42) NSCs proliferating in the presence of serum.
Figure 1B Immunocytochemical detection of Tuj1 and TN-C in clone c42 differentiated for 3 days in the absence of serum.
Figure 2 shows that Nurr1 NSCs secrete trophic and survival signals that increase the number of dopaminergic neurons in mouse primary cultures.
Figure 2A: Dissociated cells from E12.5 mouse ventral mesencephala (VM) were treated with filtered N2 media that had been conditioned by Nurr1 or control clones for 24h. Cells were fixed after 3 DIV and immunostained for tyrosine hydroxylase (TH).
Figure 2B: Total numbers of TH+ neurons in VM E12.5 primary cultures treated with conditioned media. Values represent mean + SEM of the total number of TH+ cells/cm2 of three independent experiments done in triplicate. Statistical analysis was performed by oneway ANOVA with Fisher's post hoc test (**, P<0.001; *, P<0.05).
Figure 3 shows that Nurr1 expression protects cells from oxidative stress-induced death.
Figure 3A and 3B: Nurr1 clones are resistance to oxidative-stress induced death. Control and Nurr1 clones were treated with 25µM of H2O2 for 6h and stained with propidium iodide (PI) and Hoechst 33342 and counted (n=4). Data represent the mean percent increase in cell death after 6 hours of peroxide treatment (Hoechst/PI).
Figure 3C: Fewer Nurr1-NSCs are immunoreactive for cleaved caspase-3 following exposure to oxidative stress (n=4). Statistical analysis was performed by one-way ANOVA with Fisher's post hoc test (***, P<0.0001; **, P<0.001; *, P<0.05).
Figure 4 shows that Tenascin-C null mice display premature dopaminergic differentiation in vivo.
Figure 4A: Coronal sections through E11 VM revealed that the number of DA neurons (assessed by tyrosine hydroxylase (TH) immunoreactivity) is increased in TN-C null mice when compared to control.
Figure 4B: Quantifications of TH+ cells through serial coronal sections of E11, E15, and P1 control and TN-C null mice. A correction in the number of TH+ cells is observed by E15 and at P1 in TN-C null mice.
Figure 4C: Proliferating progenitors were identified by BrdU uptake in vivo at E11 in the VM. The proliferative capacity of E11 DA precursors was not affected in TN-C null mice when compared to control animals, as assessed by immunohistochemistry against BrdU.
Figure 4D and 4E: The total number of Nurr1+ cells and radial glia (GLAST) were not altered at E11 in TN-C null mice when compared to wildtype controls. For each experiment (n=5) statistical analysis was performed using the paired t-test *P<0.05.
Figure 5A: In vitro growth kinetics of Nurr1 clones (clone 42 (c42), clone 48 (c48)) and control clones (puroB (pB), puroD (pD)). Data are shown as the mean accumulated number of viable cells ± SD of two independent cultures of the same cell lines.
Figure 5B: Venn diagram showing the distribution of the upregulated and downregulated target genes for the c42/pB and c48/pD Affymetrix chip comparisons.
Figure 5C: Functional distribution of up- and downregulated genes in common between each comparison.

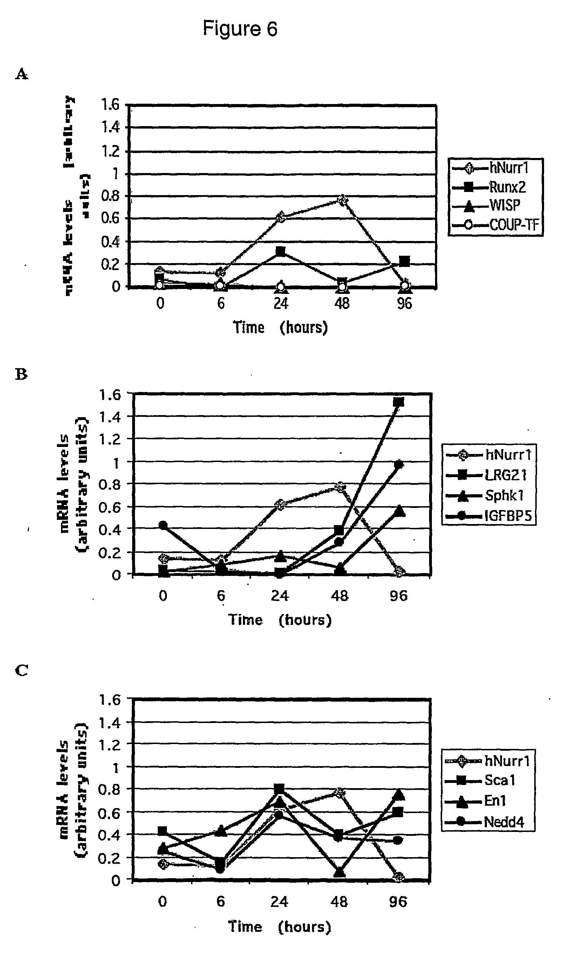

Figure 6: Temporal expression of Nurr1 responsive genes. The temporal expresson profiles of several Nurr1-regulated genes were assessed by Real-time RT-PCR on c17.2 stem cells transiently transfected with Nurr1 (A-C). mRNA levels were then analyzed at 0, 6, 12, 24, 48, and 96 hours respectively. Plots represent mRNA transcript levels of each gene normalized to control transfected cells. The HPRT housekeeping standard was used as an internal control for all experiments. With the exception of WISP and COUP-TF, modest increases in mRNA levels for Sca1, En1, Nedd4, and Runx2 and were observed at earlier time points and at 96 hours. LRG21, Sphk1, and IGFBP5 transcript levels increased at this late time point.
Figure 7: Chemokine receptors CCR1 (Figure 7A), and CCR2 (Figure 7B) are expressed in the developing midbrain
Figure 8: Both CCL2 (Figure 8A), and CCL7 (Figure 8B) are expressed in the developing midbrain.
Figure 9: Both CCL2 and CCL7 increase the number of TH positive neurons (Figure 9A, Figure 9B) and the proportion of Nurr1 precursors that acquired the expression of TH in rat ventral midbrain primary precursor cultures (Figure 9C). Interestingly, the total number of neurons (Figure 9E) or Nurr1+ precursors (Figure 9D) did not increase, indicating that CCL2 and 7 selectively increase the acquisition of a midbrain dopaminergic phenotype in Nurr1+ precursors.
Figure 10: Neither CCL2 nor CCL7 promoted proliferation or reduced cell death, as assessed by BrdU incorporation and active caspase-3 stainings.
Figure 11: Neither CCL2 (MCP1) nor CCL7 (MCP3) regulated the number of nestin+ precursors or Nurr1+ cells or TuJ1+ neurons or enhanced proliferation in neurosphere cultures in the presence of bFGF and/or EGF.
Figure 12: CCL7 promotes neuritogenesis inventral midbrain explants, indicating that this chemokine contributes to the morphological differentiation of neurons
Figure 13: Expression of CXCR2 in the developing ventral midbarin, as assessed by Q-PCR.
Figure 14: Both CXCL6 and CXCL8 increase the number of TH positive neurons but they do that by different mechanisms. CXCL8 mainly increases the number of Nurr1+ precursors (Figure 14A) and also their differentiation into TH+ (Figure 14B). Instead CXCL6 increases the number of TH+ cells by increasing the proportion of Nurr1 precursors that become TH+ (DA neurons), but without affecting the total number of Nurr1+ cells in rat ventral midbrain primary precursor cultures.
Figure 15: TUNEL staining showed no decrease in the number of positive cells in the culture after CXCL6 or CXCL8 treatment (Figure 15A). However, CXCL8, but not CXCL6, increased BrdU incorporation by three fold (Figure 15B).
Figure 16: Incorporation of BrdU was increased by two fold by CXCL8, but not by CXCL6 (Figure 16A). Although, the number of nestin positive cells in the neurospheres did not change (Figure 16B), CXCL8 increased the number of Nurr1+ cells (Figure 16C) and the number of TuJ1+ cells (Figure 16D).
Figure 17: CXCL8 increases the levels of Nurr1 mRNA in neurospheres cultured in the presence of mitogens.

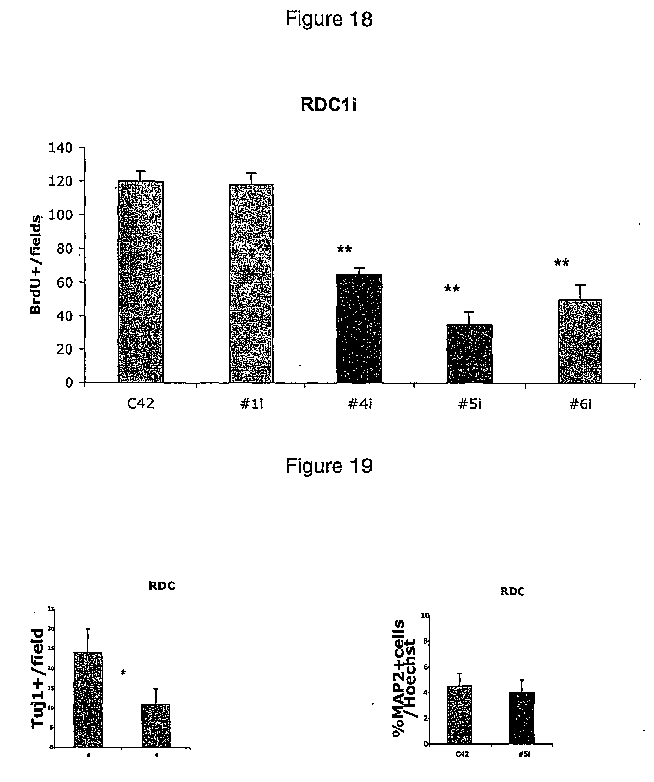

Figure 18: Decreased proliferation of a Nurr1-overexpressing c17.2 neural stem cell line (c42) after RDC1 RNAi. The figure shows different stable lines with different degrees of interference. Clone number 5i was used for subsequent experiments.
Figure 19: RNA interference of RDC1 in a Nurr1-overexpressing c17.2 neural stem cell line reduced the generation of new TuJ1+ neurons but did not affect the pool of differentiated MAP2+ neurons. The figure shows a comparison between c42 (control, green) and clone 5i (RDC1i, blue).

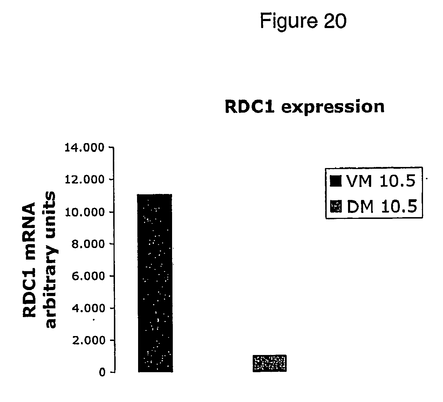

Figure 20: RDC1 is expressed at high levels in the developing ventral midbrain at E10.5, as assessed by Q-PCR.
Figure 21 shows that Nurr1 expression correlates with RDC1 induction, as assessed by Real time RT-PCR.
Figure 21A shows that RDC1 mRNA levels in Nurr1 clones are higher than in control clones.
Figure 21B shows that RDC1 expression in the midbrain peaks ventrally at E10.5.
Figure 21C shows that Nurr1 expression is induced in the ventral midbrain at E10.5.
Figure 22 shows that chemokine treatment affects the proliferation and increases the number of DA neurons in rat E14.5 ventral midbrain primary cultures.
Figure 22A shows that treatment with the chemokines SDF-1, BLC-1, I-TAC and IL-8, but not CXCL6, increases the number of proliferating cells in VM cultures. Values represent the mean percentage ± SD of BrdU+ cells in two independent experiments of 2-3 replicates each.
Figure 22B shows that treatment with SDF-1, BLC-1 and I-TAC increases the number of DA neurons cells in VM cultures. Values represent the mean percentage ± SD of TH+ cells in two independent experiments of 2-3 replicates each.

### Table 1

### Functional classification of genes upregulated in c17.2-Nurr1 stem cells

Genes commonly activated in two independent oligonucleotide chip comparisons between c17.2 Nurr1 neural stem cells (c42 and c48) and c17.2 control neural stem cells (pB and pD) were classified according to their known or putative function. Ratios represent the increase in gene expression.

### Table 2

### Functional classification of genes downregulated in c17.2-Nurr1 stem cells

Genes commonly repressed in two independent oligonucleotide chip comparisons between c17.2 Nurr1 neural stem cells (c42 and c48) and c17.2 control neural stem cells (pB and pD) were classified according to their known or putative function. Ratios represent the decrease in gene expression.

### Table 3

### Validation of microarray data

Comparison of the expression ratio of selected genes, as calculated by Affymetrix genechip analysis and quantitative PCR. For quantitative PCR, specific primers were designed and PCR values were normalized to the amplification value of the 18S housekeeping gene. Out of 11 randomly selected genes, 10 were confirmed in independent cultures Nurr1-NSCs.

### Table 4

Chemokine gene expression profile of mouse E10.5 midbrain.

### EXPERIMENTAL AND DISCUSSION

### NURR1-REGULATED GENES IN NEURAL STEM CELLS

We compared the transcriptomes of two independent control and Nurr1-expressing neural stem cell lines (NSCs) using Affymetrix cDNA microarrays. These data reveal the regulation of genes that promote cell survival (trophic/growth factors and stress response genes), and prevent cell death (decreased caspase-3 and caspase-11 expression). We observed that conditioned media from Nurr1-expressing NSCs enhanced the survival of midbrain dopaminergic neurons in primary cultures and that Nurr1-expressing NSCs themselves were more resistant to oxidative stress. These results confirm the presence of both secreted and cell-intrinsic survival signals modulated by Nurr1 during dopaminergic differentiation. These findings are accompanied by a dynamic pattern of gene regulation that is consistent with Nurr1 promoting both the acquisition of brain-region specific identity (Engrailed-1) and neuronal differentiation (tubulin β III). Our analysis suggested an important role for tenascin-C in developing DA neurons given its high upregulation in Nurr1 clones. Analysis of tenascin-C null mice revealed an increase in the number of dopaminergic neurons (TH+) at E11.5, but not at E15, suggesting that tenascin-C normally acts to maintain Nurr1 precursors.

### MATERIALS AND METHODS

### Cell culture and treatment

c17.2 neural stem cells were maintained and passaged as previously described (Snyder et al., 1992). To generate accumulated cell growth curves, the total number of viable cells was assessed at each passage by trypan blue exclusion and 500,000 viable cells were plated. At the time of the next passage (after 2 days in vitro) the number of cells generated was determined and the ratio of cell production was multiplied by the total number of cells in the previous point of the curve. Growth data were represented by the equation *y* = *a*.*2^{sx}*, where *y* is the total number of cells, *x* is the time in days in vitro (DIV), *s* is the rate of doubling, and a is a constant (Gritti et al., 1999). For differentiation, c17.2 cells were plated on poly-D-lysine coated wells in a defined, serum-free medium (N2, consisting of a 1:1 mixture of F12 and MEM supplemented with 15 mM HEPES buffer, (insulin (5 µg/mL), apo-transferrin (100µg/mL), putrescine (100 µM), progesterone (20 nM), selenium (30 nM), glucose (6 mg/mL), bovine serum albumin (BSA, 1 mg/mL), and grown for 3DIV. Cells were fixed with 4% paraformaldehyde (PFA) for 20 minutes prior to immunocytochemical analysis. For primary cultures, ventral mesencephala from E12.5 mouse embryos were dissected, treated with dispase/DNase, mechanically dissociated, and plated at a density of 125,000 cells/cm2 in N2 media supplemented with 20 ng/mL bFGF for 2 hours. Media was then changed to 0.2 µm filtered N2 that had been conditioned by c17.2 cells for 24 hours.

### RNA preparation, real-time RT-PCR, and quantification of gene expression

RNA from c17.2 cells was extracted using RNeasy Mini Kit (Qiagen). 5 µg of total RNA from two control (pB and pD) and two Nurr1-overexpressing cell lines (c42 and c48) was treated with RQ1 RNase-free DNase (Promega, Madison, WI) and RNA integrity was assessed by electrophoresis. 1 µg of RNA was reverse transcribed using SuperScript II Reverse Transcriptase (Invitrogen) and random primers (Invitrogen) (RT+ reaction), and parallel reactions without reverse transcriptase enzyme were done as a control (RT- reaction). Quantitative PCR was performed as previously described (Castelo-Branco et al., 2003).

### High density oligonucleotide array hybridization

RNA probes were labeled according to the supplier's instructions (Affymetrix, Santa Clara, CA, USA). First strand synthesis was carried out by a T7-(dT)24 primer and SuperScript II Reverse Transcriptase (Gibco BRL Life Technologies) using 10 µg of total RNA sample. Second strand synthesis was performed according to the SuperScript Choice System (Gibco BRL Life Technologies) by *E*. *coli* DNA-Polymerase I, *E*. *coli* Ligase, and RNaseH. Fragment end-polishing was performed using T4-Polymerase. An in vitro transcription reaction was used to incorporate Biotin-11-CTP and Biotin-16-UTP into the cRNA probe (BioArray HighYield RNA Transcript Labeling Kit, Enzo). The fragmented cRNA was hybridized overnight (45°C) to the DNA chips. Washes were done in the GeneChip Fluidics Station (Affymetrix) according to the manufacturer's protocol. Staining was performed with RPhycoerythrin Streptavidin (Molecular Probes), followed by an antibody amplification procedure using biotinylated anti-streptavidin antibody (Vector laboratories) and goat IgG (Sigma). The scanning was carried out at 3 µm resolution, 488 nm excitation- and 570 nm emission-wavelength using the GeneArray Scanner (Hewlett Packard). Two control (pB and pD) and two Nurr1-overexpressing (c42 and c48) cell lines were processed independently. Data were scaled based on total intensity and data analysis was performed with the Affymetrix software (MAS 5.0). The p-values were calculated by applying the Wilcoxon's signed rank test and then used to generate the complex calls. Complete microarray data was registered at the NCBI Gene Expression Omnibus (GEO) and hybridization array data repository as GSE3571 (http://www.ncbi.nlm.nih.gov/geo/ - website findable using any web browser).

### Immunocytochemical analysis

Cultures were blocked for 1 hour at room temperature in PBST (1x PBS, 1% BSA, and 0.3% Triton X-100) and overnight at 4°C with the corresponding primary antibody diluted in blocking buffer. The following antibodies were used: mouse anti-β-tubulin type-III (TuJ1), 1:2000 (Sigma); mouse anti-TH, 1:10,000 (Diasorin); rabbit anti-GFAP, 1:500 (Dako); rabbit anti-tenascin-C (TN-C), 1:250 (Chemicon); mouse anti-cleaved caspase-3 (Asp175), 1:100 (Cell Signaling Technology); mouse Nestin (Rat-401), 1:500 (DSHB, University of Iowa). After washing, cultures were incubated with biotinylated secondary antibodies diluted 1:500 for 2 hours and immunostaining was visualized using Vector Laboratory ABC immunoperoxidase kit and Supergrey substrate. For immunofluorescence, 1:100 dilutions of Cy2- or Rhodamine-coupled secondary antibodies (Jackson ImmunoResearch) were used. Cultures were then rinsed twice in PBS and analyzed.

### Cell death and oxidative stress assays

For oxidative stress experiments, c17.2 cells were grown to 60% confluency, plated at a density of 5000 cells/cm², and allowed to grow overnight. The following day, fresh media was added, and cells were treated with freshly diluted 25 µM" H₂O₂ for 0 or 6 hours. Following peroxide treatment, cells were fixed with 4% PFA for 20 minutes, rinsed, and blocked with PBS/5% goat serum for 30 minutes at room temperature. Immunocytochemistry for cleaved caspase-3 was performed as described above. Next a 1:500 dilution of Hoechst 33328 (0.5 mg/ml) was added for 5 minutes at RT. Cells were washed twice in PBS, visualized under a Zeiss HBO100 microscope, and counted. For cell viability assays, cell lines were grown, plated, and treated as described above. Cells were stained with 100 µg/ml Hoechst 33342 and 2 µg/ml of propidium iodide in ice cold PBS. After 10 minutes, the unfixed, stained cells were immediately counted.

### Tenascin-C knockout mice and immunohistochemistry

Tenascin-C (TN-C) null mice were previously described (Fukamauchi et al., 1997; Fukamauchi et al., 1996). Embryos were fixed in ice-cold 4% PFA for 6 hours, cryoprotected in 20% sucrose, and frozen in OCT compound at -70°C. Next, serial coronal 14 µM sections of the entire ventral midbrain were cut on a cryostat. Immunohistochemistry was performed on 4% paraformaldehyde-postfixed slides. Incubations were carried out at 4°C overnight with the following antibodies diluted in PBS, pH 7.3, 1% BSA, and 0.3% Triton X-100 solution: rabbit anti-Nurrl (1:500 dilution, Santa Cruz Biotech.), rabbit anti-TH (1:1000, Pel-Freeze), rat anti-BrdU (1:200, Abcam), mouse anti-phosphorylated histone H3 (Ser10) (1:100, Upstate), mouse anti-RC2 (1:200, DSHB), guinea pig anti-glutamate transporter, GLAST (1:2000, Chemicon). After washes, the sections were blocked for 30 minutes in dilution buffer and then incubated for 2-4 hours with a secondary antibody [Cy2/3 (cyanine)-coupled donkey anti-mouse IgG, Cy2-coupled donkey anti-rabbit IgG (1:200 dilution, Jackson ImmunoResearch). Immunostaining was visualized by using a Zeiss HBO100 microscope. For BrdU analysis, pregnant mice were injected with 0.5 ml of a 1 mg/ml BrdU/saline solution 6 hours before sacrifice. Pretreatment with 2 N HCl for 15 minutes prior to preincubation with primary antibody was needed for detection of BrdU. Stainings were visualized using a Zeiss HBO100 microscope and images were collected with a Hamamatsu camera C4742-95.

### RESULTS

### Microarray analysis of Nurr1 neural stem cells.

While overexpression of Nurr1 facilitates the differentiation of neural stem cells (Wagner et al., 1999) and ES cells (Chung et al., 2002; Kim et al., 2002) into midbrain dopaminergic (DA) neurons, the effects of Nurr1 on downstream gene expression in a proliferating cell have been unclear.

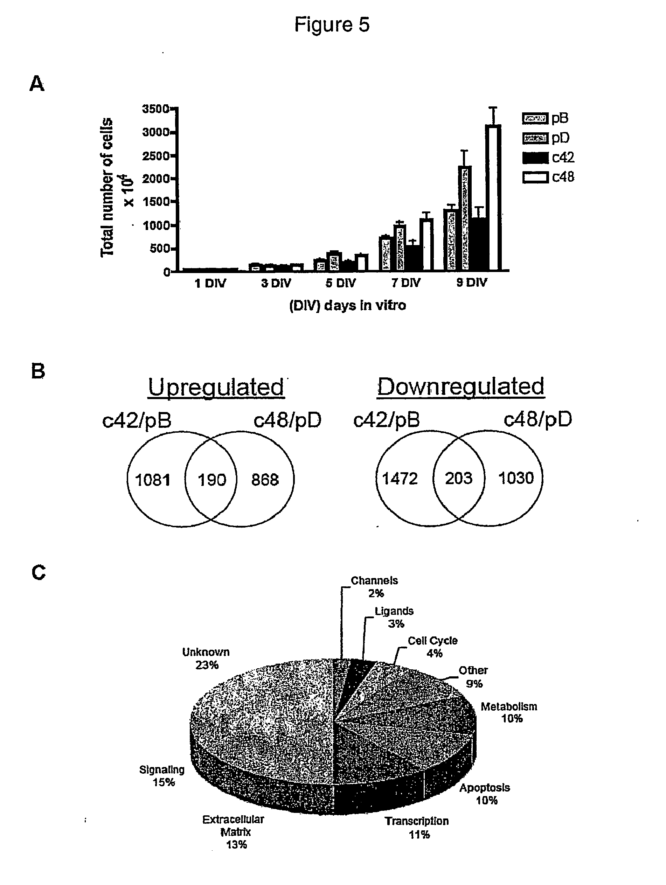

We determined the transcription profile of proliferating c17.2 neural stem cell lines stably transfected with Nurr1 (Wagner et al., 1999). Two Nurr1-overexpressing clones, clone 42 and clone 48, and two control clones transfected with a control vector, pB and pD, were chosen for this study. Clone 42 and clone 48 were selected among all the Nurr1-transfected cell lines generated due to their high potential to become TH+ dopaminergic neurons upon co-culture with immature ventral mesencephalic glia (Wagner et al., 1999). Accumulated in vitro cell growth curves were generated (Figure 5A), and population doubling rates (PoD, the reciprocal of the doubling time in DIV) for the clones were estimated from these curves (PoD=0.57 ± 0.05 for clone 42, PoD=0.84 ± 0.02 for clone 48, PoD=0.53 ± 0.09 for clone pB, PoD=0.75 ± 0.01 for clone pD).

In order to minimize the effects of proliferation on the study, the Nurr1 and corresponding control clones were matched (c42 with pB and c48 with pB) according to their doubling rate and further processed for gene expression comparison (Figure 5A).

The overall gene expression pattern of clone 42 (c42) *versus* clone puroB (pB) and of clone 48 (c48) *versus* clone puroD (pD) was compared using the Affymetrix murine U74v2 type A microarray. Total RNA from the c17.2 clones was isolated, reverse transcribed, and hybridized to the chip. Of the >12,000 genes queried, a total of 1949 genes were upregulated (1081 in the c42/pB subtraction, and 868 in the c48/pD subtraction), whereas 2502 genes were downregulated (1472 in c42/pB and 1030 in c48/pD). However, only those genes regulated in a coordinate manner between the two independent comparisons were considered for further validation and quantitative analysis. These genes included 190 activated and 203 repressed genes (Figure 5B).

### Classification and confirmation of genes regulated by Nurr1 in neural stem cells

The distribution of identified genes with known or putative functions is shown in Figure 5C and a complete list is available online (see NCBI/GEO GSE3571). The highest number of genes up- and downregulated represented expressed sequence tags (23%) whose functions are unknown. The majority of characterized genes regulated encoded transcription factors (11%) and proteins involved in intracellular signaling (15%). The expression of several secreted ligand (3%) inducing growth factors, and extracellular matrix/cell adhesion molecules (13%) was also regulated. In addition, Nurr1 overexpression downregulated a number of cell cycle genes (4%) including cyclin B2, D1, and p21, whose regulation has been implicated in the differentiation of ventral progenitors (Joseph et al., 2003). Nurr1 overexpression also resulted in the upregulation of trophic/survival signals that coincided with the downregulation of a number of pro-apoptotic genes (10%) (downregulation of caspase-3 and -11). For a more detailed analysis, we focused our studies on those genes either up-or downregulated at least 2-fold or an average of 2.5-fold in both of the two independent comparisons, and classified them by broad biological function (see Table 1 and Table 2, respectively). Many genes were represented several times in the chip and showed concordant expression, thereby validating the hybridization. To confirm the results of the microarray, genes were randomly selected for quantitative PCR analysis either in the respective clones or in c17.2 neural stem cells transiently transfected with Nurr1. We found that in virtually all cases analyzed (10 out of 11) the selected genes were corroborated (Table 3).

Additionally, to independently assess whether the gene regulation found in Nurr1 clones was truly associated with Nurr1 overexpression, transient transfection experiments of the parental c17.2 neural stem cell line were also performed. The expression profiles of 9 selected genes that were regulated in the microarray were analyzed at several time points (Figure 6).

According to our data, 7 out of these 9 genes also displayed higher levels of expression upon Nurr1 transient transfection, and most remained activated at 96h post-transfection that is, after the transient expression peak detected for Nurr1 mRNA.

### Nurr1 induces the expression of neuronal markers in NSCs

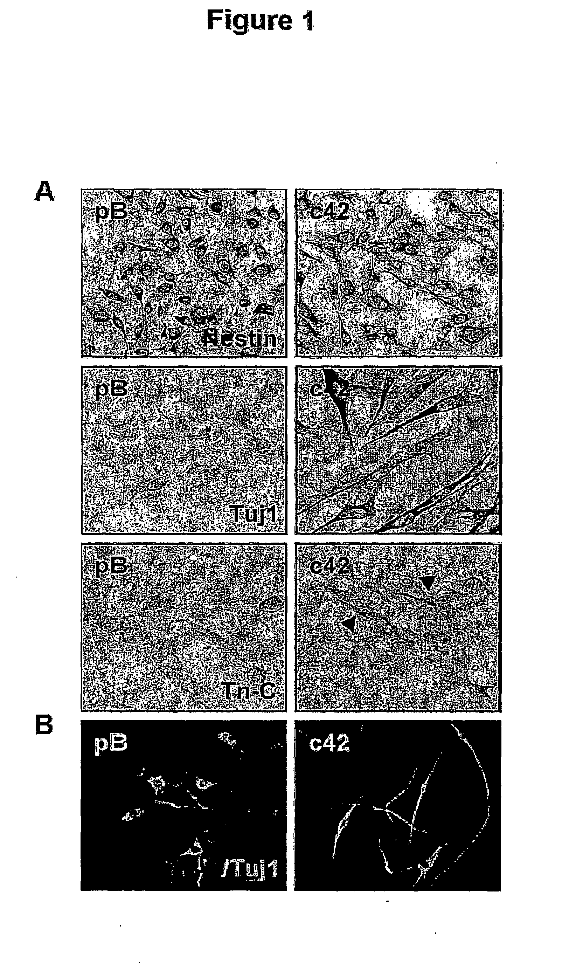

Several lines of evidence, including our microarray, have suggested distinct functions for Nurr1 in neuronal differentiation (Arenas, 2002; Arenas, 2005). To investigate the effects of Nurr1 in this process, we stained both control and Nurr1 NSC clones with several markers that define stages of progressive neuronal differentiation. Both control and Nurr1 NSCs stained positive for the neural stem cell marker Nestin (Figure 1A). However, only Nurr1-overexpressing clones exhibited high levels of immunoreactivity for the neuronal cytoskeletal protein tubulin P III (Tuj1) (Figure 1A), a gene upregulated 4-fold in the microarray. Nurr1-overexpressing NSCs were also immunoreactive for tenascin-C, an extracellular matrix protein critical for the development and maintenance of several neuronal types (Fukamauchi et al., 1997; Fukamauchi et al., 1996) (Figure 1A). Consistent with these observations was the finding that virtually all Nurr1-overexpressing cells, but not control cells, acquired a bipolar neuronal morphology, and were double-labeled with antibodies against Tuj1 and tenascin-C upon differentiation in defined media without mitogens (Figure 1B). Similar results were observed with c48 and pD. These results confirm that elevated Nurr1 levels in undifferentiated neural stem cells activate the expression of several genes (tubulin β III, neurofilament, and TN-C), that define a neuronal phenotype as identified by the genechip analysis.

### Nurr1 overexpression promotes survival and resistance to oxidative stress

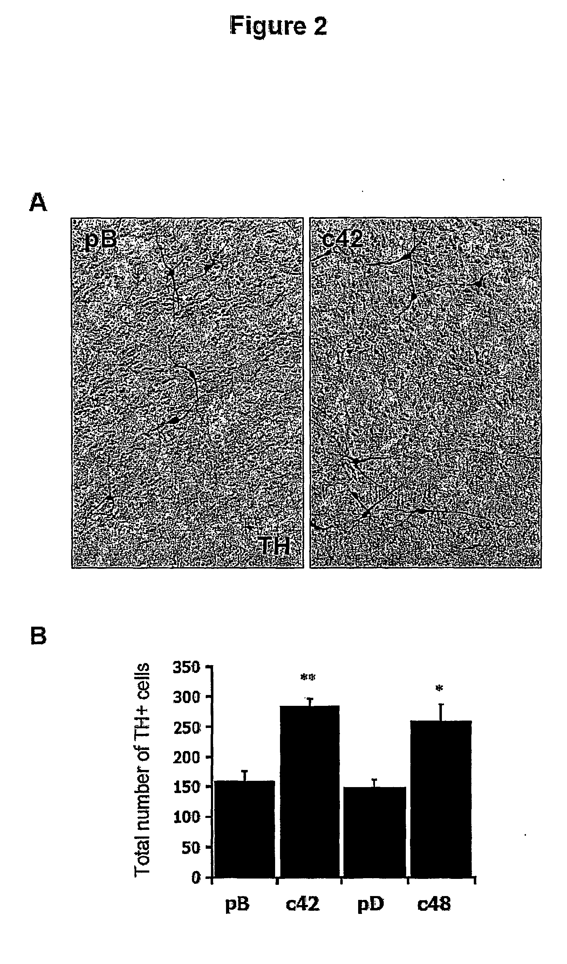

The results of the genechip revealed several trophic factors and survival signals upregulated by Nurr1, including TGF-! and NGF (upregulated at least 2-fold in both clones).
To assess whether the trophic factors expressed by Nurr1-overexpressing cells were biologically active, conditioned media from control and Nurr1-overexpressing cell lines was collected. Addition of conditioned media from c42 or c48 cells to ventral mesencephalic E12.5 primary cultures increased the number of TH+ dopaminergic neurons compared to the addition of conditioned media from both control cell lines (Figure 2A and 2B), suggesting that the trophic factors expressed by Nurr1 clones indeed promote the survival and/or differentiation of DA neurons.

In addition to secreted factors, several survival, stress response/DNA repair, and antiapoptotic factors were also induced (e.g., LRG-21, gadd45β, and PKC)# in the Nurr1 clones.

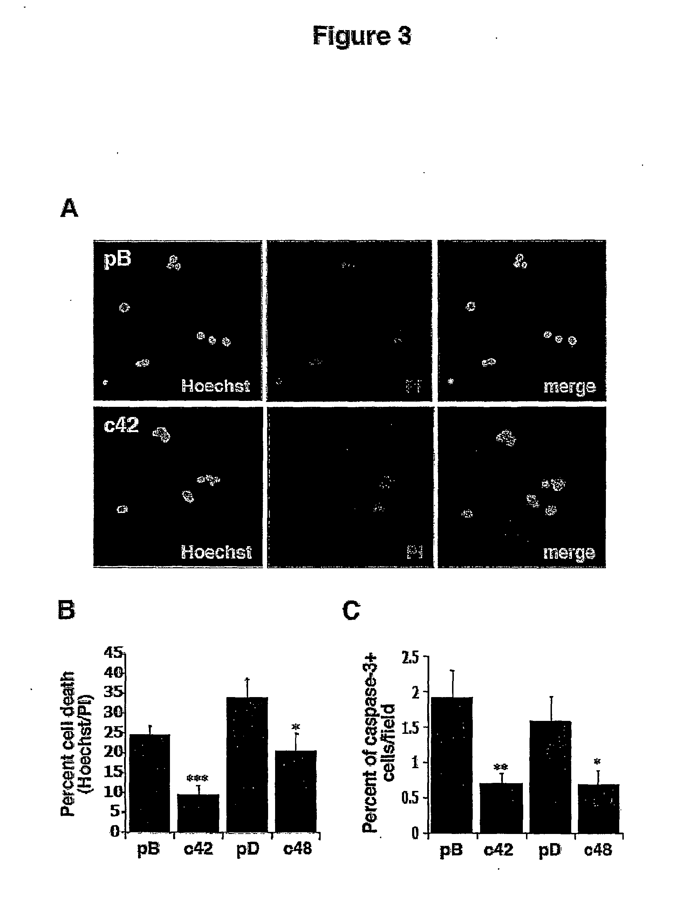

Notably, a number of pro-apoptotic genes were downregulated (e.g., ASM-like phosphodiesterase, Mekk1, caspase-3, and caspase-11). An upregulation of several genes
(e.g., oxidative stress-induced protein and cytochrome b5)
involved in regulating the cellular response to oxidative stress was also observed (upregulated approximately 1.5-fold in both clones). Oxidative stress has been implicated in the pathophysiology of several neurodegenerative diseases including PD (Jenner and Olanow, 1996; Klein and Ackerman, 2003; Sherer and Greenamyre, 2005). We therefore examined whether Nurr1 expression protected cells from death induced by oxidative stress. Both sets of Nurr1-overexpressing neural stem cells and controls were treated with several concentrations of H2O2 and viable cells were counted after 6h. Peroxide treatments above 25µM H2O2 were effective at inducing cell death in both sets of clones. Cultures from both sets of control and Nurr1 overexpressing cells were treated with 25µM peroxide and then stained with propidium iodide to identify dead cells. Significantly (40%) less cell death was observed in cells overexpressing Nurr1 compared to controls (Figure 3A and 3B). To confirm the extent of apoptosis upon peroxide treatment, we assessed the presence of cleaved caspase-3 by immunocytochemistry.
Fewer Nurr1-overexpressing cells were immunoreactive for cleaved caspase-3 compared to controls (Figure 3C). Together, these results demonstrate that Nurr1 promotes cell survival and helps confer resistance to oxidative stress on NSCs by downregulating several proteins involved in the apoptotic response.

### Premature differentiation of DA neurons in tenascin-C null mice

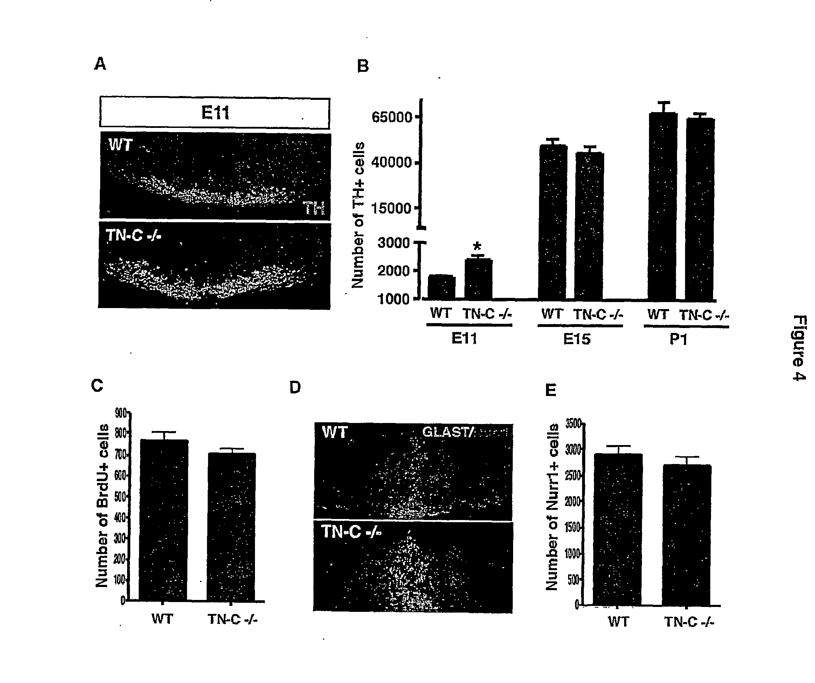

TN-C expression has been previously detected throughout the ventral mesencephalon from E9-E13. However, the role of TN-C in the development of dopaminergic neurons remains largely unknown (Kawano et al., 1995). Our gene expression profiles suggested an important role for TN-C in developing DA neurons given its upregulation in Nurr1 clones (Table 1). To investigate the role of TN-C in vivo, we analyzed tenascin-C knockout mice. At the onset of dopaminergic neurogenesis (E11), the number of tyrosine hydroxylase immunoreactive cells was increased in TN-C null mice (Figure 4A and 4B). This increase was particularly confined to the more lateral dopaminergic neuron population. However, the excess of dopaminergic neurons was corrected for by E15 and P1, a time period where neurogenesis has ended in vivo (Figure 4B). We hypothesized that the early phenotype observed in TN-C knockouts could reflect an overproduction of proliferating DA progenitors or a change in the proliferative capacity of the VM. To assess the later, we compared the number of proliferating cells between genotypes using BrdU, a marker that labels cells in Sphase of the cell cycle. BrdU analysis revealed no change in the number of proliferating cells in the ventral mesencephalon at E11 between the wildtype and TN-C null mice (767.66 ± 59.88 and 708.00 ± 37.32, respectively Figure 4C) or E12.5. These results were further confirmed using an additional marker of proliferating cellos, phosphorylated histone H3. The number of active caspase-3 immunoreactive cells did not differ between wildtype and TN-C null mice. These data suggest that deletion of TN-C does not increase the number of DA neurons by altering proliferation or cell survival. We next examined, radial glia, a cell that can act as a stem cell and direct regionspecific neural differentiation in the central nervous system (Haubensak et al., 2004; Sherer and Greenamyre, 2005) including the developing midbrain (Hall et. al., unpublished data). However, no change in RC2 or GLAST immunoreactivity was observed in TN-C null mice at E11 (Figure 4D). In addition, TN-C knockout mice were analyzed for changes in Nurr1+ DA precursors. At E11, no alterations in Nurr1+ cells were observed between wildtype and TN-C knockout mice (Figure 4D and 4E). These data indicate that TNC normally acts to maintain early Nurr1+ precursors in a TH- state in vivo and that upon its deletion, the DA differentiation process is accelerated at E11, but the total number of TH+ cells at the end of neurogenesis is not altered.

### DISCUSSION

In order to gain more insights into the molecular pathways regulated by Nurr1 during development, we performed an analysis of genes regulated by Nurr1 in a proliferating neural stem cell line using Affymetrix microarrays. Of more than 12,000 genes queried, we identified approximately 400 genes that showed regulation by Nurr1. The findings we report here support a role for Nurr1 in reducing the potential of a neural stem/precursor cell by initiating its differentiation into a neuronal phenotype.

Interestingly, this process was accompanied by an increase in the expression of genes that provide regional identity in the brain and confer cell survival and oxidative stress resistance. Thus, Nurr1 appears to coordinate the function of diverse genes during development.

The role of Nurr1 in promoting stem cell differentiation is supported by data from this microarray indicating that expression of neural stem cell markers, mitogens, and their cognate receptors, and proteins that control cell cycle progression are downregulated (see NCBI/GEO GSE3571). The downregulation of these genes may contribute to the coupling of cell cycle exit and differentiation programs in Nurr1+ cells. Interestingly, Nurr1 induces cell cycle arrest and post-mitotic maturation in cooperation with the cyclin dependent kinase inhibitor p57kip2 in the dopamine-synthesizing neuronal cell line MN9D (Castro et al., 2001). Accordingly, we have found several cell cycle genes downregulated in our study, in particular, Cyclin B2, D1, and p21 (all downregulated at least 1.5-fold in both comparisons and confirmed by quantitative PCR).

Our results suggest that proliferating c17.2-Nurr1 cells represent a transition state from the multipotent neural stem cell to a precursor/immature neuron. Accordingly, the cytoskeletal protein encoding the neurofilament medium polypeptide, was strongly induced in the Nurr1 clones. This result, together with the co-localization of tubulin β III (an early neuronal marker) and tenascin-C (involved in neurogenesis) in Nurr1 clones, suggest that proliferating c17.2-Nurr1 cells have already initiated a neuronal differentiation program. A strong repression of B-FABP expression was also detected; a fatty acid transporter that is highly expressed in neuroepithelial and radial glia cells in the developing brain and enriched in NSC cultures (Conti et al., 2005; Kurtz et al., 1994). Accordingly, the expression of several genes involved in non-neuronal development was repressed, such as the muscle cytoskeletal protein Myosin X (downregulated 2.5-fold in both clones). Together, these results indicate that Nurr1 expression has positive effects on the development of a neuronal phenotype in NSCs.

Another important set of genes controlled by Nurr1 in a coordinated manner are those involved in promoting cell survival. Several studies have identified proteins critical for the induction and survival of mesencephalic dopaminergic neurons (Lin and Rosenthal, 2003; Smidt et al., 2003).

Furthermore, RXR ligands that activate Nurr1-RXR heterodimers have been demonstrated to be critical for the survival of dopaminergic neurons (Wallen-Mackenzie et al., 2003). Here, we observed an upregulation of several neurotrophic factors and downregulation of pro-apoptotic genes in Nurr1-overexpressing NSCs. Among the genes upregulated in Nurr1-overexpressing NSCs were two trophic signals required for the induction (TGF-β) and postnatal development of dopaminergic neurons (GDNF) (Farkas et al., 2003; Granholm et al., 2000). In fact, both TGF-β and GDNF have been shown to alleviate functional deficits in animal models of PD (Fernandez-Espejo et al., 2003; Gill et al., 2003). Consistent with an upregulation of trophic factors was the observation that conditioned media from c42 or c48 cells increased the number of TH+ dopaminergic neurons in ventral mesencephalic E12.5 primary cultures compared to control conditioned media. Our results suggest that Nurr1-overexpression induces the expression of several signals later required for the survival of dopaminergic neurons.

Moreover, both survival and stress response/DNA repair/anti-apoptotic factors were induced (e.g., LRG-21 and gadd45p, both upregulated 2.5- and 1.6-fold respectively) while pro-apoptotic genes were downregulated (e.g., caspase-3, and caspase-11) in Nurr1 clones.

Another gene regulated by Nurr1 in c17.2 cells was LRG-21, a member of the activating transcription factor/CREB protein family that is involved in the cellular stress response (Hai and Hartman, 2001) and is regulated by seizures (Chen et al., 1996) and axotomy (Tsujino et al., 2000). We also detected increased levels of gadd45p, an inducible factor that belongs to a protein family associated with cell-cycle control and DNA repair (Zhan et al., 1999), and downregulates pro-apoptotic c-Jun N-terminal kinase signaling (De Smaele et al., 2001).

Thus, combined, our results indicate that one of the main functions of Nurr1 is to promote survival by orchestrating the expression of stress response and anti-apoptotic genes. To further investigate the prosurvival effects of Nurr1, we examined whether Nurr1 expression protected cells from death induced by oxidative stress. The potential for oxidative damage is intrinsically associated with Nurr1+/TH+ dopaminergic neurons, since tyrosine hydroxylase activity forms oxygen radicals in a redox mechanism with its cofactor (Adams et al., 1997). Cell survival upon oxidative treatments above 25µM H₂O₂ was greater for Nurr1 clones compared to controls. Microarray data, indicating an upregulation in the expression of several oxidative-stress proteins important for maintaining free radical homeostasis in Nurr1-overexpressing cells (see NCBI/GEO GSE3571), also supported these findings. Nurr1-NSCs also exhibited lower levels of cleaved caspase-3 protein after peroxide treatment, compared to controls. This result is in agreement with previous findings showing that deletion of one Nurr1 allele decreases the survival of DA neurons in vitro (Eells et al., 2002) and that decreased Nurr1 expression increases the vulnerability of DA neurons to MPTP, a neurotoxin that induces oxidative stress and PD-like symptoms in rodents (Imam et al., 2005; Le et al., 1999; Sherer and Greenamyre, 2005). Nurr1 clones also expressed lower levels of caspase-11.

Recent reports have indicated that caspase-11 knockout mice are more resistant to MPTPmediated cell death than wild-type mice (Furuya et al., 2004). Moreover, the recent identification of Nurr1 mutations in PD patients (Le et al., 2003) further suggests that the expression of survival and/or cell death genes regulated by Nurr1 could be altered in PD. One of the highest induction rates in proliferating c17.2 Nurr1-NSCs corresponded to tenascin-C, an extracellular matrix glycoprotein. Tenascin-C is differentially expressed in specific patterns throughout nervous system development. Tenascin-C expression correlates well with the migration, differentiation, and decreased proliferation of oligodendrocyte precursor cells, as well as axonal growth and guidance in neurons (Garcion et al., 2001; Joester and Faissner, 2001). Interestingly, adult mice lacking TN-C also exhibit hyperlocomotive activities that have been attributed to altered levels of tyrosine hydroxylase and dopamine (Fukamauchi et al., 1997). However, the observed increase in locomotor activity cannot be attributed to increased DA cell number since TN-C null mice display higher numbers of DA neurons at the onset, but not at the end, of DA neurogenesis. Our results suggest the occurrence of a premature differentiation of Nurr1+ precursors in TN-C null mice at E11 that normalizes by E15. These data are in agreement with other findings showing normal phenotypes elsewhere later in development (Garcion et al., 2001; Garwood et al., 2004). Our data also indicate that at the onset of neurogenesis, TN-C does not regulate the size of the proliferative progenitor pool but contributes instead to preserve Nurr1+ postmitotic precursors in a TH- state. Recent reports have described TN-C as a regulator of Wnt signalling (Beiter et al., 2005). Studies from our laboratory have also shown that Wnt signals and betacatenin overexpression differentially regulates the maintenance and conversion of Nurr1+ precursors into DA neurons (Castelo-Branco et al., 2004; Castelo-Branco et al., 2003). Thus our results highlight a novel function for TN-C to maintain early Nurr1+ postmitotic precursors in an undifferentiated state. Blocking or inhibiting TN-C may be used to accelerate the generation of large numbers of DA neurons in vitro for cell replacement therapies.

### REFERENCES

Adams et al. (1997) Redox Rep 3, 273-9.

Arenas, E. (2002) Brain Res Bull 57, 795-888.

Arenas, E. (2005) Ann N Y Acad Sci 1049, 51-66

Beiter et al. (2005) beta-Catenin regulates the expression of tenascin-C in human colorectal tumors. Oncogene

Castelo-Branco et al. (2004) J Cell Sci 117, 5731-7. Castelo-Branco et al. (2003) Proc Natl Acad Sci U S A 100, 12747-52.

Castillo et al. (1998) Mol Cell Neurosci 11, 36-46.

Castro et al. (2001) J Biol Chem 276, 43277-84.

Chen et al. (1996) Mol Cell Biol 16, 1157-68.

Chung et al. (2002) Eur J Neurosci 16, 1829-38.

Conti et al. (2005) PLoS Biol 3, e283.

Crispino et al. (1998) Brain Res Mol Brain Res 59, 178-88. 23

De Smaele et al. (2001) Nature 414, 308-13.

Eells et al. (2002) Behav Brain Res 136, 267-75.

Farkas et al. (2003). J Neurosci 23, 5178-86.

Fernandez-Espejo et al. (2003) Rev Neurol 36, 540-4. Fukamauchi et al. (1997) Biochem Biophys Res Commun 231, 356-9.

Fukamauchi et al. (1996) Biochem Biophys Res Commun 221, 151-6.

Furuya et al. (2004) J Neurosci 24, 1865-72.

Garcion et al. (2001) Development 128, 2485-96.

Garwood et al. (2004) Eur J Neurosci 20, 2524-40.

Gill et al. (2003) Nat Med 9, 589-95.

Granholm et al. (2000) J Neurosci 20, 3182-90.

Gritti et al. (1999) J Neurosci 19, 3287-97.
25

Hai et al. (2001). Gene 273, 1-11.

Hall et al. (2003) Glia 43, 47-51.

Haubensak et al. (2004) Proc Natl Acad Sci U S A 101, 3196-201.

Imam et al. (2005) Faseb J 19, 1441-50.

Jenner et al. (1996) Neurology 47, S161-70.

Joester et al. (2001) Matrix Biol 20, 13-22.

Joseph et al. (2003) Proc Natl Acad Sci U S A 100, 15619-24.

Kawano et al. (1995) Brain Res Dev Brain Res 86, 101-13.

Kim et al (2002) Nature 418, 50-6.

Klein et al. (2003). J Clin Invest 111, 785-93.

Kurtz et al. (1994) Development 120, 2637-49.

Le et al. (1999) J Neurochem 73, 2218-21.

Le et al. (2003) Nat Genet 33, 85-9.

Lin et al. (2003) Semin Cell Dev Biol 14, 175-80. 27

Maruyama et al. (1998) Int J Oncol 12, 1237-43.

Saucedo-Cardenas et al. (1998) Proc Natl Acad Sci U S A 95, 4013-8.

Sherer et al. (2005) Antioxid Redox Signal 7, 627-9.

Smidt et al. (2003) Eur J Pharmacol 480, 75-88.

Snyder et al. (1992) Cell 68, 33-51.

Tsujino et al. (2000) Mol Cell Neurosci 15, 170-82.

Wagner et al. (1999) Nat Biotechnol 17, 653-9.

Wallen et al. (1999) Exp Cell Res 253, 737-46.

Wallen-Mackenzie et al. (2003) Genes Dev 17, 3036-47.

Zetterstrom et al. (1997) Science 276, 248-50.

Zetterstrom et al. (1996) Brain Res Mol Brain Res 41, 111-20.

Zhan et al. (1999) Oncogene 18, 2892-900.

*THE* β-*CHEMOKINES CCL2 AND CCL7 REGULATE THE DIFFERENTIATION OF NURR1+ VENTRAL MIDBRAIN PRECURSORS INTO DOPAMINERGIC NEURONS*

Chemokines (chemotactic cytokines) constitute a family of small protein ligands that regulate diverse functions in cells of the immune system (Rossi and Zlotnic, 2000). Chemokines are classified into four major groups (based on where cystein residues are located), referred to as C, CC, CXC and CX3C. The CC family, also known as the β-chemokine family, have no amino residue intervening between the first two conserved cysteines (Bajetto et al., 2000).
Chemokines and their receptors are expressed by all major cell types in the CNS (neurons, astrocytes and glia) and were initially recognized as playing a role in the leukocyte migration and communication, both in physiological and pathophysilogical contexts (Bajetto et al., 2001). Chemokine receptors belong to the large family of of seven-transmembrane domain receptors which couple to heterotrimeric GTP-binding proteins (G-proteins). Although there are many chemokine receptors, most of them are rather promiscuous and bind not only to one but several ligands (Gether and Koilka, 1998; Wilson and Bergsma, 2001).
The β-Chemokines are best known for its function in inflammatory processes, with cellular sites on astrocytes, perivascular microglia and infiltrating leukocytes (Fang 2000; Murdock and Finn 2000; Baggiolini 2001). The β-Chemokines differs from the other chemokines by being very potent chemoattractants and activators for monocytes (Baggiolini et al., 1997). In addition, a growing body of evidence suggests that chemokines and their receptors also mediate intercellular communication in the central nervous system (CNS) (Bajetto et al., 2001). In agreement with this view, CCR2, a chemokine receptor that binds to both CCL2 and CCL7, is expressed by neurons and glia in the rat brain (Jun Feng Ji et al., 2004). Moreover, Banisadr et al. (2002 and 2005) found that CCR2 is expressed in the adult substantia nigra and using fluorescent immunohistochemistry they localized the expression of CCR2 in dopaminergic (DA) neurons of the substantia nigra pars compacta. These findings suggested that β-Chemokines such as CCL2 and CCL7, if expressed in the midbrain or their targets, they may signal to dopaminergic neurons. Interestingly, we have previously shown by microarray analysis and Q-PCR that CCL7 is upregulated (Sousa et al, unpublished) in a neural stem cell line overexpressing Nurr1 that is capable of differentiating into DA neurons (Wagner et al., 1999). These results suggested that CCL7 may also play a role in the development of DA neurons.

We hereby report that both CCL2 and CCL7 are expressed at high levels in the developing ventral midbrain (VM) compared to the dorsal midbrain. We also found that the β-chemokine receptors CCR1 and CCR2 are expressed in VM precursors. Moreover, when rat E14.5 VM primary precursor cultures were treated with CCL2 or CCL7, an increase in the number of tyrosine hydroxylase (TH) neurons and in the proportion of TH+/Nurr1+ cells were detected. Provided that neither CCL2 nor CCL7 had any effect on the number of Tuj1, Nurr1 or BrdU positive cells or the survival of the cells, our results indicate that CCL2 and CCL7 promote the development of DA neurons by selectively enhancing the differentiation of Nurr1+ precursors into TH+ DA neurons.

### MATERIAL AND METHODS:

### Chemokine array

2.5µg of total RNA was retrotranscribed to cDNA using SuperScript II Reverse Transcriptase (Gibco BRL Life Technologies) and ³²P dCTP. Probes were hybridized to the mouse chemokine GEQArrays (Superassay) and the data were analyzed according to the manufacturers recommendations.

### Real time RT-PCR and quantification of gene expression

Total RNA was isolated from ventral midbrain of mice embryonic day (E) 10.5, 11.5, 13.5, 15.5 and P1. Reverse transcription reaction and real time RT-PCR was carried out as described (Castelo-Branco et al. 2003). Quantum RNA classical 18S internal standard was purchased from Ambion (Austin, USA) and PCR primers from DNA Technology a/S Aarhus, Denmark. The following PCR programme was used for SYBR Green detection on the ABI PRISM 5700 Detection System (PE Applied Biosystems, Foster City, CA, USA): 94°C for 2 minutes; 35-40 cycles of 94°C for 30 seconds, 60°C for 30 seconds, 72°C for 15 seconds; and 80°C for 5 seconds. Statistical analysis of the results was performed using a two-tailed Wilcoxson Signed rank test: Significance for all tests was assumed at the level of P<0.05(*P<0.05,
**0.01<P<0.001; ***P<0.001).

### Precursor cultures and treatments

VM from 14.5 embryos obtained from time-mated Sprague-Dawley rats, were dissected, mechanically dissociated, and plated at a final density of 1x10⁵ cells/cm² on poly-D lysine coated 6- or 24-well plates in N2 serum free medium, consisting of a 1:1 mixture of F12 and MEM with N2 supplement, 300mg glucose, 50mg BSA, 750 microliter HEPES and 250 microliter glutamine. All factors were added once, at the initiation of culture and BrdU was added 6 hours prior to fixation.

### Neurosphere cultures and treatments

VMs were dissected from E11.5 wild-type CD-1 mice embryos, dissociated using protease and DNase, and plated at a final density of 100.000 cells/ml in 35 mm dishes. The cells were cultivated in complete Medium: 41.5 ml F12 and 41.4 ml DMEM with 3 ml glucose (20%), 1.5 ml NaHCO3 (7.5%), 0.5 ml 1M hepes, 1 ml L-glutamine, 1 ml gentamicin, 50 µl heparin, 0.4 g BSA and 10 ml Hormone mix (1:1 mixture of F12 and DMEM, 1.2 ml glucose (20%), 0.6 ml NaHCO3 (7.5%), 0.2 ml hepes (1M), 40 mg apo-t-transferine, 10 mg insulin, puteserine, progesterone and sodium selenite). bFGF and EGF was added at a concentration of 20 ng/ml each with/without 300 ng/ml CCL2 and 100 ng/ml CCL7 (R & D Systems). For long-term cultures, neurospheres were collected after 4 days by centrifugation, re-suspended in complete medium, and triturated using a 200µl automatic pipette and plated as described above.

### Immunocytochemical analysis

Cells were fixed for immunocytochemistry in ice-cold 4% paraformaldehyde for 15-20 minutes and washed with PBS and incubated over night in dilution buffer 1xPBS, 1% BSA, and 0.3% Triton-X 100) with one of the following antibodies:
mouse ∝TH (1:250 dilution; Immunostar), rabbit ∝TH (1:500; Pelfreeze), rabbit ∝Nurrl (1:500; Santa Cruz), rabbit ∝active caspase III (1:100; Cell Signalling), mouse ∝BrdU (1:200; Dako), rat ∝BrdU (1:200; Abcam), Tuj1, or Nestin. After washing, cultures were incubated for1-2 hours in dilution buffer with the appropriate secondary antibody/ies (biotinilated 1:500, CY2 or rhodamine coupled horse-anti-mouse IgG 1:100, all from vector). Bright field immunostaining was visualized with the Vector Laboratory ABC immunoperoxidase kit, using NovaRed (red), SG or 3-3' diaminobenzidine tetrahydrocloride (DAB 0.5 mg/ml)/nickel chloride (1.6 mg/ml) (gray/black), or VIP (violet) substrates. Double immunostaining was performed by sequential single staining as described above. At the end of all staining procedures, cultures were incubated with Hoechst 33258 reagent for 10 minutes. BrdU immunocytochemistry included incubation for 30 minutes with 2M HCL. Images were acquired from stained cells in PBS at room temperature with a Zeiss Axioplan 100M microscope (LD Achrostigmat 20X, 0.3 PHI ∝0.2 and LD Achroplan 40X, 0.60 Korr PH2 ∝0.2) and collected with a Hamamatsu camera C4742.95 (with QED imaging software).

### Immunoblotting

Tissue was collected from mouse E11.5 ventral midbrain and was lysed in lysis buffer. The protein was transferred to a clean tube for protein measurement using the BCA kit (pierce) and stored in Laemmli buffer. Equal amounts of protein were analyzed by polyacrylamide gel electrophoresis (12.5% polyacrylamide). The proteins were transferred onto a polyvinylidene diflouride membrane. After blocking in PBS with 5% milk for 1 hour, the membrane was incubated with the following primary antibodies over night at 4°C: goat α-MCP-1/JE (1:500; RnD); goat α-MCP-3/MARC (1:250; RnD). After wasing, the membrane was incubated with alkaline phosphatase-conjugated secondary α-goat antibody (1:5000; Pharmacia Amersham) for 1 hour at room temperature and subsequently developed according to manufacturer's instructions for enhanced chemiluminescence.

### Statistical analysis

Quantitative immunocytochemical data represent the means ± s.e.m. of counts from ten non-overlapping fields, in three wells per condition from three separate experiments. For the RT-PCR experiments, three separate experiments were analyzed. Statistical analysis was performed in Prism4 (GraphPad software, San Diego, USA) as described in the figure legends, with significance for all tests assumed at the level of P<0.05 (*P<0.05, **0.01<P<0.001; ***P<0.001). Statistical tests were chosen according to the distribution of the sample population.

### RESULTS

### Chemokine array

We performed a genechip analysis using mRNA extracted from mouse embryonic ventral midbrain (VM) and dorsal midbrain (DM) tissue in developmental stages, E10.5 and E12.5. The mRNA was retrotranscribed to ³²P-labelled cDNA and was hybridized to a Gearray membrane containing 67 chemokines and chemokine receptor genes. Out of these 67 different genes, we found 14 genes to be expressed in the developing midbrain (see Table). We focused our attention on ligands belonging to the β-chemokine family (CC-family). In the E10.5 midbrain, one β-chemokine (CCL7) was expressed at higher level in the VM, compared to DM. The same chemokine was also expressed at a later stage, at E12.5, in the VM.

### Expression of CCR receptors in the ventral midbrain

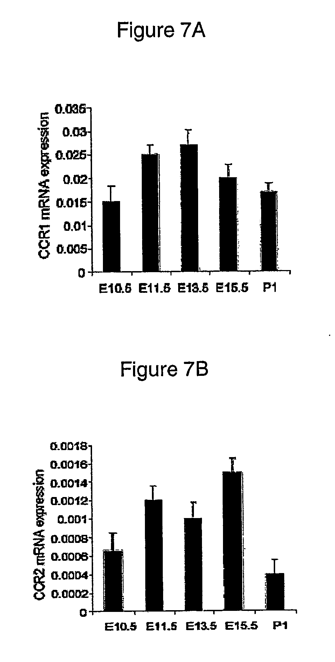

Chemokine receptors belong to a large family of seven-transmembrane domain receptors which couple to heterotrimeric GTP-binding proteins (G-proteins). Although there are different kinds of chemokine receptors, most of them can bind several ligands (Gether and Koilka, 1998; Wilson and Bergsma, 2001). However, every ligand binds to a specific subset of receptors. For instance, while CCL7 binds to CCR1, CCR2, CCR3, and CCR9, another ligand, CCL2, binds to CCR2 and CCR11. We next performed quantitative real time PCR analysis (Q-PCR) and found that both CCR1 and CCR2 are expressed at different stages in mouse VM (E10.5, E11.5 E13.5, E15.5, and P1) and are developmentally regulated (Figure 7A-B).

### Expression pattern of CCL2 and CCL7

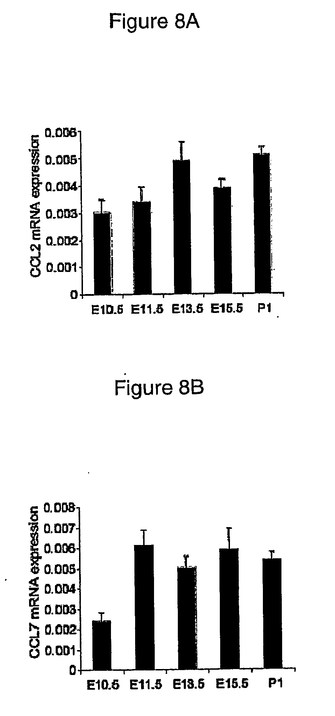

In the developing ventral midbrain, tyrosine hydroxylase (TH) dopaminergic neurons are born around embryonic day E11.5 in mice. Prior to the birth of TH+ neurons in this brain region, the transcription factor and orphan nuclear receptor Nurr1 is expressed by dopaminergic cell precursors, at E10.5. Nurr1 is required for proper dopaminergic neuron development (Zetterstrom et al., 1997; Saucedo-Cardenas et al., 1998; Castillo et al., 1998). We have previously demonstrated that Nurr1 overexpression induces neuronal differentiation of the c17.2 neural stem cell line and that these cells adopt a dopaminergic fate upon co-culture with embryonic/newborn midbrain astrocytes (Wagner et al., 1998). We have also recently found that CCL7 is upregulated in a Nurr1 overexpressing neural stem cell line (Sousa et al., in preparation). We therefore decided to examine by Q-PCR whether CCL7 and CCL2 are expressed in the developing mouse VM. We examined E10.5, E11.5, E13.5, E15.5 and P1 stages and found that CCL7 and CCL2 are developmentally regulated (Figure 8A-B). Moreover, analysis of protein expression by western blot revealed detectable levels of both CCL7 and CCL2. Thus, our results indicate that CCL7, CCL2 and their receptor CCR2 are expressed in the developing ventral midbrain at the time of birth of dopaminergic neurons. We therefore decide to examine whether CCL7 can also regulate the development of VM DA neurons.

### CCL7 and CCL2 promote the differentiation of Nurr1+/TH-precursors into Nurr1+/TH+ neurons in ventral midbrain precursor cultures

To elucidate the function of CCL7 in dopaminergic development, we performed rat E14.5 primary precursor cultures and compared its effects to that of CCL2.

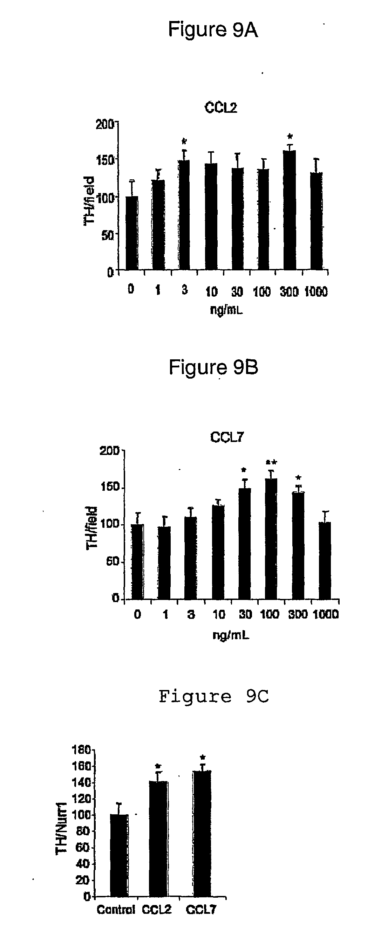

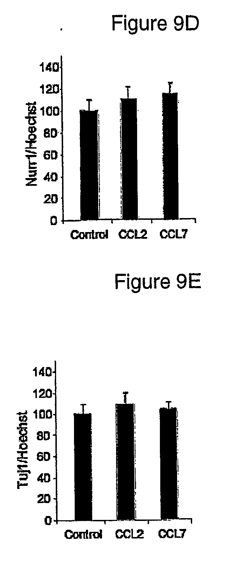

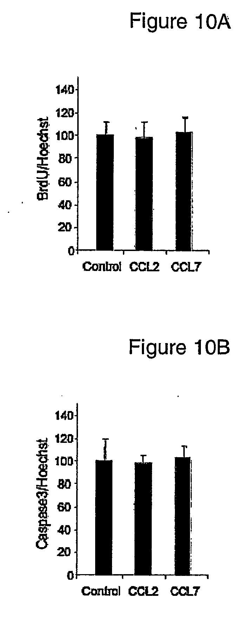

We examined the number of cells expressing Nurr1 or tyrosine hydroxylase (TH), two markers of DA precursors and DA neurons. Dose response analysis showed that CCL7 and CCL2 increased the number of TH+ cells in a dose-dependent manner, reaching maximal effects at 0.1 □g/ml CCL7 and 0.3 □g/ml CCL2 (Figure 9A-B). We also detected an increased proportion of TH+/Nurr1+ cells in rat VM primary cultures (Figure 9C). Given that neither CCL7 nor CCL2 had any effect on the number of Tuj1, Nurr1 (Figure 9D and 9E) or BrdU positive cells or the survival of the cells (Figure 10A and 10B), our results indicate that CCL2 and CCL7 selectively promote the differentiation of Nurr1+ precursor into TH+ neurons.

### CCL2 and CCL7 do not promote proliferation of ventral midbrain neurospheres but induce neuritogenesis in explant cultures

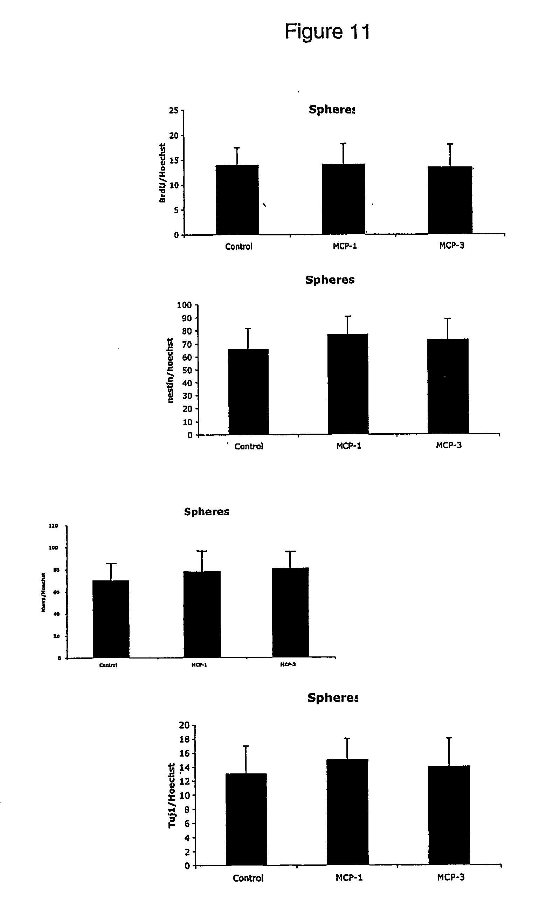

Next we examined whether CCL2 and CCL7 could also contribute to the proliferation of VM precursors in neurosphere cultures, in the presence of bFGF and EGF. We found that neither of the two β-chemokines regulated the proliferation of precursors as assessed by BrdU incorporation (Figure 11A). Moreover, we found that in this culture condition the number of nestin+ precursors did not change (Figure 11B) and the number of newborn TuJ1+ neurons was not different from control (Figure 11C). These results indicate that CCL7 and 2 are devoided of mitogenic effect and that depite their pro-differentiation activity, they can not overcome the effect of the mitogens.

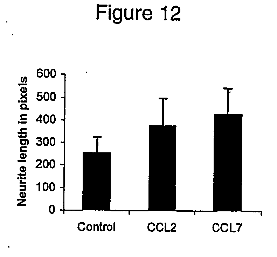

We next examined whether CCL2 and CCL7 could also work to promote the migration of neurons and neuritogenesis in VM explant cultures. We found found that CCL7 induced a significant increase (2 fold) in migration and in neurite length (Figure 12). Thus, combined, our results indicate that CCL7 induces the morphological differntiation of neurons, but it can not induce the differentiation of VM precursors in the presence of mitogens.

### REFERENCES

Baggiolini M. Chemokines in pathology and medicine. J. Intern. Med. 2001; 250, 91-104.

Banisadr et al. J. Neurochem. 2002; 81: 257-269.

Bajetto et al. Front Neuroendocrin. 2001 Jul; 22(3): 147-84.

Bajetto et al. J. Neurochem. 202 Sep; 82(6): 1311-29.

Feng et al. Immunology. 2000; 21: 203-210.

Gether et al. J. Biol. Chem. 1998 Jul; 273(29): 17979-17982.

Jun Feng et al. Neuroscience letters. 2004; 355: 236-240.

Murdoch et al. Blood. 2000; 95: 3030-3043.

Rossi et al. Immunol. 2002; 18: 217-42.

### THE α-CHEMOKINES CXCL6 AND CXCL8 DISTINCTIVELY REGULATE PROLIFERATION AND DOPAMINERGIC DIFFERENTIATION OF VENTRAL MIDBRAIN PRECURSORS AND NEUROSPHERES

Increasing evidence suggests that α-chemokines serve several important functions in the nervous system including regulating neuroimmune responses, modulating neurotransmission, and promoting neuronal survival and CNS development. In this study we examined the expression and function of two α-chemokines, CXCL6 and CXCL8, and their receptors in the developing midbrain. We found that both α-chemokines and one of their receptors, CXCR2, are developmentally regulated during ventral midbrain development. Moreover, while CXCL6 and 8 promoted the differentiation of Nurr1+ precursors into dopaminergic neurons in vitro, only CXCL8 enhanced their proliferation. The finding that CXCL8 regulated both proliferation and differentiation of VM precursors prompted us to examine whether CXCL8 could be applied to expand and differentiate ventral midbrain neurospheres. Our results show that this is the case and suggest that α-chemokines may find an application in cell replacement therapy for Parkinson's diseases, as a tool to enhance the number of dopaminergic neurons in precursor/stem cell preparatations.

Chemokines (chemotactic cytokines) constitute a family of small protein ligands that regulate diverse functions in cells of the immune system (Rossi and Zlotnik, 2000). Chemokines are classified into four major groups (based on where cystein residues are located), referred to as C, CC, CXC and CX3C. The CXC members, also called alfa-chemokine subfamily, have a single aminoacid residue intervening between the first two conserved cystein residues (Bajetto et al., 2000). Additionally, based on the presence or absence of a glutamine acid-leucine-arginin motif immediately preceding the first cystein, the CXC chemokines are further divided in two subclasses, ELR versus non-ELR. The presence of this tripeptide is necessary for the interactions with neutrophil chemokine receptors (Herbert et al., 1991).

Chemokines and their receptors were initially recognized as playing a role in leukocyte migration and communication, both in physiological and pathophysiological contexts. These molecules control immune cell trafficking and recirculation of the leukocyte population between the blood vessels, lymph, lymphoid tissues and organs, a process important in the host immune surveillance, and in acute and chronic inflamatory responses (Fang, 2000; Murdock and Finn, 2000; Baggiolini, 2001), including HIV-1 infection (Alkhatib et al., 1996). Surprisingly, however, chemokines and their receptors are expressed by all major cell types in the CNS (neurons, astrocytes and oligodendrocytes), and a growing body of evidence suggests that chemokines and their receptors also mediate intracellular communication in the central nervous system (CNS) (Bajetto et al., 2001). For instance, the CXCL1/CXCR2 signaling is involved in patterning of the spinal cord by controlling the positioning of oligodendrocyte precursors (Tsai et al., 2002). Also, the CXC ligand SDF-1 and its cognate receptor CXCR4 are required for normal development of the hippocampal dental gyrus (Lu et al., 2002; Bagri et al., 2002) and are implicated in proliferation of cerebellar granule cell precursors in cooperation with Sonic hedgehog (Klein et al., 2001). Moreover, the formation of Cerebellar granule cell layer is abolished in mice lacking either SDF-1 (Ma et al., 1998) or CXCR4 (Ma et al., 1998; Zou et al., 1998), and SDF-1 also works as a guidance molecule for cerebellar granule neurons (Arakawa et al., 2003). Similarly, SDF1 and CXCR2 have been recently reported to guide axonal growth (Lieberam et al., 2005). Therefore, CXC chemokines and CXCR receptors regulate diverse mechanisms and functions such as proliferation, migration, and maturation of several subtypes of precursors during CNS development.

Up to date, chemokines have not been involved in the development of dopaminergic neurons. However, other cytokines such as interleukin 1b and interleukin 11 had been reported to regulate and promote dopaminergic neuron development. We decided to investigate whether two α-chemokines, CXCL6 and CXCL8, and their receptors, CXCR1 and 2, regulate midbrain development. We first found that CXCL6, CXCL8 and CXCR2, but not CXCR1, are expressed and developmentally regulated in the ventral midbrain. We then examined whether these chemokines regulated proliferation, survival and differentiation of ventral mesencephalic dopaminergic precursors in primary and neurospheres cultures. Our results show that CXCL6 promotes the differentiation of Nurr1+ precursors into TH+ neurons, and that CXCL8 increases both the proliferation of DA progenitors and their differentiation into dopaminergic neurons.

### MATERIAL AND METHODS

### Real time RT-PCR and quantification of gene expression

Total RNA was isolated from embryonic day (E) 14.5 VM precursor cultures (2.5*10⁶ cells in 6 cm² dishes treated with recombinant human IL-6 and recombinant human IL-8 for three days in vitro. Total RNA was also collected from E 11.5 VM neurosphere cultures, treated as above, after 6 days in vitro. Reverse transcription reaction and real time RT-PCR was carried out as described (Castelo-Branco et al. 2003). Quantum RNA classical 18S internal standard was purchased from Ambion (Austin, USA) and PCR primers from DNA Technology a/S Aarhus, Denmark. The following PCR programme was used for SYBR Green detection on the ABI PRISM 5700 Detection System (PE Applied Biosystems, Foster City, CA, USA): 94°C for 2 minutes; 35-40 cycles of 94°C for 30 seconds, 59°C for 30 seconds, 72°C for 15 seconds; and 80°C for 5 seconds. Statistical analysis of the results was performed using a two-tailed Wilcoxson Signed rank test:
Significance for all tests was assumed at the level of P<0.05(*P<0.05, **0.01<P<0.001; ***P<0.001).

### Immunohistochemistry on Brain Sections

Male and female wild-type CD-1 mice (25-35 g; Charles River Breeding Laboratories) were housed, bred, and treated according to the guidelines of the European Communities Council (directive 86/609/EEC) and the Society for Neuroscience (available at www.sfr.org/handbook, or findable using any web browser), and all experiments were approved by the local ethical committee. Mice from embryonic day (E) 10.5 and E11.5 were removed and paraformaldehyde-postfixed for 4 hours. The embryos were then imbedded in 20% sucrose over night before rapidly frozen in OCT (optimal cutting temperature) compound at -70°C. Serial coronal sections (14 µm thick) were collected on microscope slides (StarFrost, Friedrichsdorf, Germany). Incubations were carried out at 4°C overnight with mouse ∝CXCR2 (1:100 dilution; R & D Systems) and rabbit anti-Nurr1 (1:200 dilution; Santa Cruz Biotechnology) in dilution buffer (PBS, pH 7.3/1% BSA/0.3% Triton X-100). After washes with 0.2% Tween 20/PBS, the sections were blocked for 30 min in dilution buffer and then incubated for 2 h with a secondary antibody [Cy2 (cyanine)-coupled horse anti-mouse IgG, Cy2-coupled horse anti-rabbit IgG, or rhodamine-coupled horse anti-mouse IgG; 1:200 dilution; Jackson ImmunoResearch). Immunostaining was visualized by using a Zeiss HB0100 microscope.

### Precursor cultures and treatments

VM from 14.5 embryos obtained from time-mated Sprague-Dawley rats, were dissected, mechanically dissociated, and plated at a final density of 1x10⁵ cells/cm² on poly-Dlysine coated 6- or 24-well plates in N2 serum free medium, consisting of a 1:1 mixture of F12 and DMEM with 10 ng/ml insulin, 100 µg/ml transferrin, 100 µM putrescine, 20nM progesterone, 30nM selenium, 6 mg/ml glucose, and 1 mg/ml BSA. All factors were added once, at the initiation of culture and BrdU was added 6 hours prior to fixation.

### Neurosphere cultures and treatments

VM from 11.5 embryos obtained from wild-type CD-1 mice, were dissected, dissociated using protease and DNase, and plated at a final density of 50.000 cells/ml in 35 mm dishes in Complete Medium (41.5 ml F12 and 41.4 ml DMEM with 3 ml glucose (20%), 1.5 ml NaHC03 (7.5%), 0.5 ml 1M hepes, 1 ml L-glutamine, 1 ml gentamicin, 50 µl heparin, 0.4 g BSA and 10 ml Hormone mix (1:1 mixture of F12 and DMEM, 1.2 ml glucose (20%), o.6 ml NaHCO3 (7.5%), 0.2 ml hepes (1M), 40 mg apo-t-transferine, 10 mg insulin, puteserine, progesterone and sodium selenite) bFGF and EGF was added at a concentration of 20 ng/ml each with/without 500 ng/ml IL-6 and 2000 ng/ml IL-8 (R & D Systems). For long-term cultures, neurospheres were collected after 6-7 days by centrifugation, re-suspended in complete medium, and triturated using a 200µl automatic pipette and plated as described above.

### Immunocytochemical analysis

Cells were fixed for immunocytochemistry in ice-cold 4% paraformaldehyde for 15-20 minutes and washed with PBS and incubated over night, or 1 hour at room temperature in dilution buffer (1xPBS, 1% BSA, and 0.3% Triton-X 100)with one of the following antibodies: mouse ∝TH (1:1000 dilution; Immunostar): rabbit ∝TH (1:250; Pelfreeze): rabbit ∝Nurr1 (1:000; Santa Cruz): rabbit ∝active caspase III (1:100; Cell Signalling): mouse ∝BrdU (1:100; Dako: rat ∝BrdU (1:100; Abcam): after washing, cultures were incubated for1-2 hours in dilution buffer with the appropriate secondary antibody/ies (biotinilated 1:500, CY2, rhodamine coupled horse-anti-mouse IgG 1:100, all from vector). Bright field immunostaining was visualized with the Vector Laboratory ABC immunoperoxidase kit, using NovaRed (red), SG or 3-3' diaminobenzidine terrahydrochloride (DAB 0.5 mg/ml)/nickel chloride (1.6 mg/ml) (gray/black), or VIP (violet) substrates. Double staining was performed by sequential single staining as described above. At the end of all staining procedures, cultures were incubated with Hoechst 33258 reagent for 10 minutes. BrdU immunocytochemistry included incubation for 30 minutes with 2M HCL. Images were aquired from stained cells in PBS at room temperature with a Zeiss Axioplan 100M microscope (LD Achrostigmat 20X, 0.3 PHI ∝0.2 and LD Achroplan 40X, 0.60 Korr PH2 ∝0.2) and collected with a Hamamatsu camera C4742.95 (with QED imaging software).

### Statistical analysis

Quantative immunocytochemical data represent the means ±s.e.m. of counts from ten non-overlapping fields, in three wells per condition from three separate experiments. For the RT-PCR experiments, three separate experiments were analyzed. Statistical analysis was performed in StatView, as described in the figure legends, with significance for all tests assumed at the level of P<0.05 (*P<0.05, **0.01<P<0.001; ***P<0.001). Statistical tests were chosen according to the distribution of the sample population.

### RESULTS

### Expression of CXCR receptors in the ventral midbrain

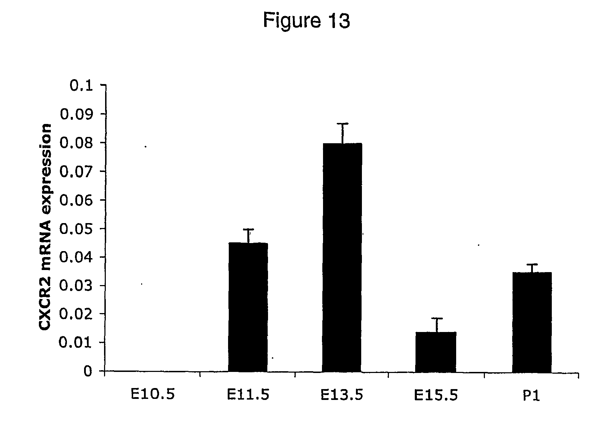

Interleukin 6 and 8 both share the two receptors; CXCR1 and CXCR2. In mouse, CXCR1 do not have a functional homologue. However, CXCR2 is expressed in the developing mouse brain (Figure 13).

### CXCL6 and 8 promote the differentiation of Nurr1+/TH-

### precursors into Nurr1+/TH+ neurons in ventral midbrain precursor cultures

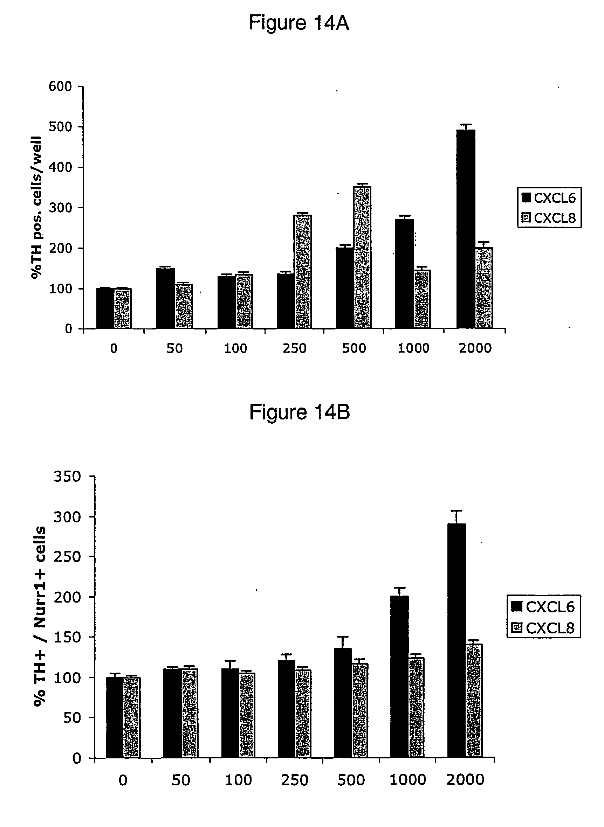

In order to examine the function of CXCL6 and 8 in ventral midbrain dopaminergic neuron development, we performed rat E14.5 primary precursor cultures and examined the number of cells expressing Nurr1 or TH, two markers of dopaminergic precursors and DA neurons. Dose response analysis showed that CXCL6 and CXCL8 increased the number of Nurr1+ cells in a dose-dependent manner, reaching maximal effects at 0.5 µg/ml CXCL8 and 2 µg/ml CXCL6 (Figure 14A). A similar dose response was observed for TH+ DA neurons, which showed a 3.5 and 4.5 fold increase after treatment with CXCL8 and CXCL6, respectively (Figure 14B). In order to examine the mechanism by which the number of TH neurons increased in the culture we first examined whether CXCL6 and 8 enhanced the differentiation of dopaminergic precursors as assessed by the conversion of Nurr1+/TH- cells into Nurr1+/TH+ cells. Interestingly, we found that both CXCL6 and CXCL8 promoted the differentiation of dopaminergic precursors into dopaminergic neurons in a dose-dependent manner, reaching maximal effects at 2 µg/ml (Figure C). Thus, our results indicate that α-chemokines promote dopaminergic differentiation in VM precursor cultures.

### CXCL8 promotes proliferation in ventral midbrain precursor cultures

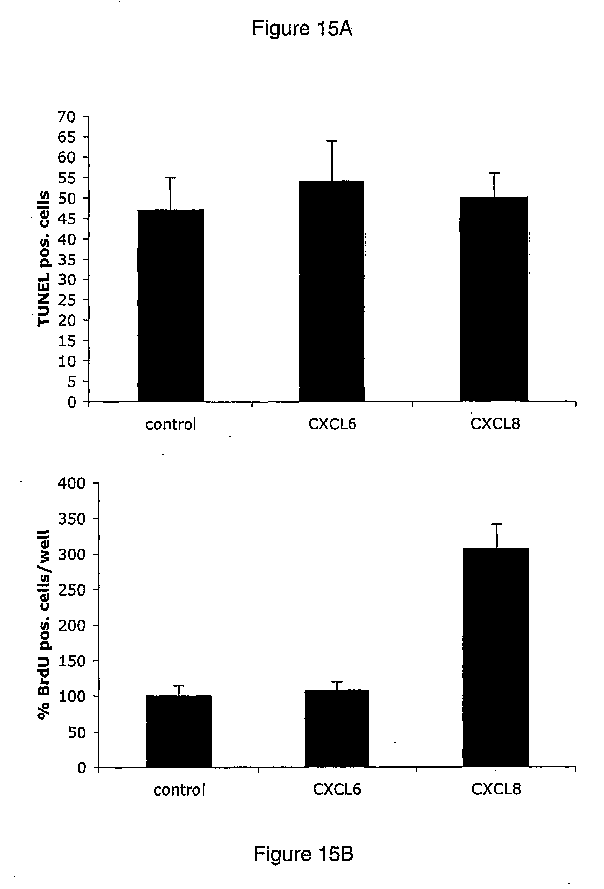

We next examined whether CXCL6 and CXCL8 in addition of regulating the differtiaion of DA precursors could also regualte the proliferation and survival the cultures. TUNEL staining showed no decrease in the number of positive cells in the culture after CXCL6 or CXCL8 reatment (Figure 15A), indicating that none of the two chemokines exherted a survival-promoting effect. However, when BrdU incorporation was analysed, we found that CXCL8, but not CXCL6, increased by three fold the number of proliferating cells (Figure 15B). Thus, our results show that CXCL8 promotes the proliferation of precursors in the the culture and that both CXCL6 and CXCL8 promote the differentiation of precursors into dopaminergic neurons.

### CXCL8 promotes the proliferation and differentiation of ventral midbrain neurospheres

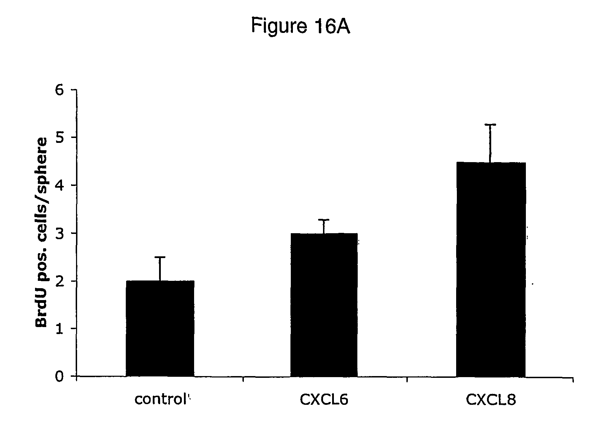

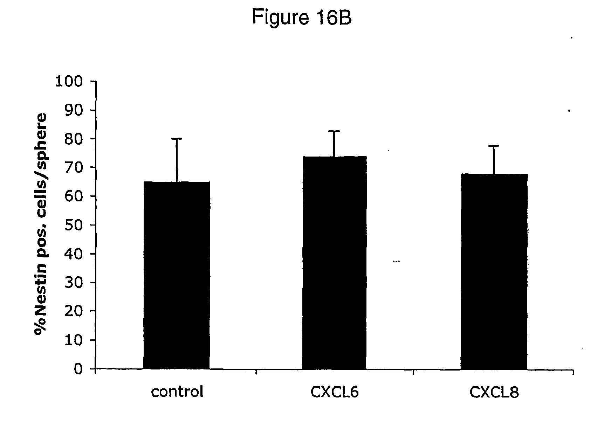

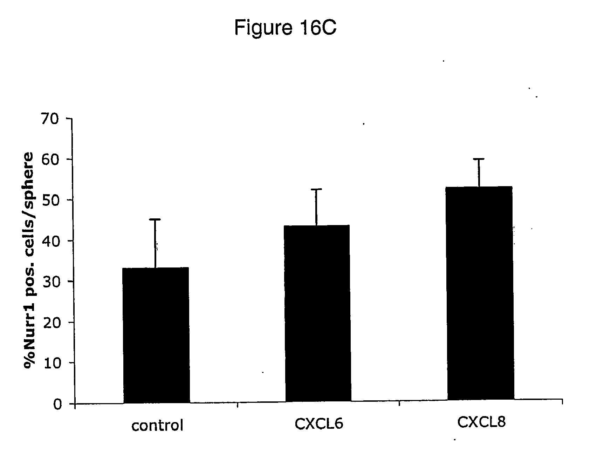

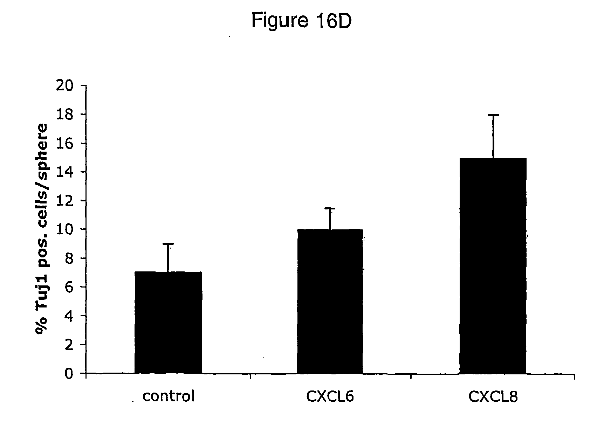

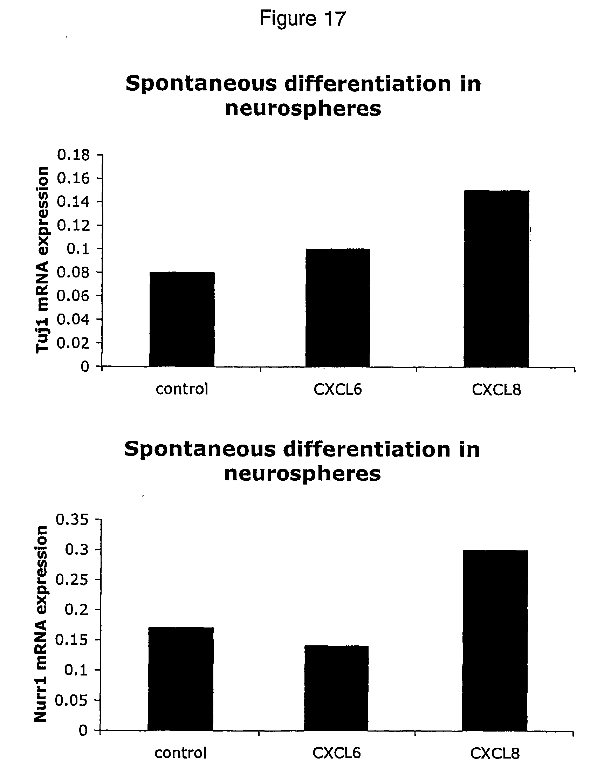

We next examined whether CXCL6 and CXCL8 could also be used to promote the proliferation and differntiation of VM neurospheres. Mice E10.5 ventral midbrain neurospheres were grown in bFGF, Shh and FGF8. As shown in primary precursor cultures, incorporation of BrdU was increased by two fold by CXCL8, but not by CXCL6 (Figure 16A), indicating that CXCL8 indeed exherts a clear mitogenic effect on VM neurosphere cultures. Importantly, the number of nestin positive cells in the neurospheres (Figure 16B) and the number of spheres did not change, indicating that the effect was not on self-renewal but on proliferation of VM porgenitors. In this culture condition, CXCL8 also increased the number of Nurr1+ cells (Figure 16C) and the number of TuJ1+ cells (Figure 16D). Moreover, we also found that CXCL8 increased the expression of beta III tubulin and Nurr1 mRNA (Figure 17) as assessed by Q-PCR in these cultures. These results indicate that neuronal differentiation takes place in these cultures.

### CXCL6 promotes the dopaminergic differentiation of ventral midbrain neurospheres

Next we examined whether CXCL6 could also contribute to the differentiation of neurospheres and explant cultures. We found that CXCL6 promoted the differentiation of clone 42 Nurr1 neural stem cells grown in the presence of bFGF as neurospheres and had a marked neuritogenic effect on DA neurons of rat E14.5 VM explants. Thus our results indicate that CXCL6 promotes the morphological differentiation of neural stem and progenitors cells. These findings suggest that CXCL6 may be used to enhance neuronal differentiation in preparations containing dopaminergic neurons.

### REFERENCES

Alkhatib et al. Virology. 1997 Aug 4;234(2):340-8.

Baggiolini et al. J. Intern. Med. 2001; 250,91-104.

Bajetto et al. Front Neuroendocrin. 2001 Jul;22(3):147-84.

Bajetto et al. 2002 Sep;82(6):1311-29.

Banisadr et al. European Journal of Neuroscience. 2002;16,1661-1671.

Feng Immunology. 2000;21,203-210.

Gavreli et al. J. Cell Biol. 1992; 119,493-501.

Gether et al. JBC. 1998 Jul 273(29): 17979-17982. Review.

Haass et al. Nature. 2000 Mar 23;404:341-343. Review.

Lu et al. Proc Natl Acad Sci U S A. 2002 May 14;99(10):7090-95.

Mennicken et al. Trends Pharmacol. Sci. 1999;20,73-78

Murdoch et al. Blood.2000; 95,3030-3043.

Rossi et al. Annu Rev immunol. 2002;18:217-42. Review.

Shimizu et al. Journal of virology. 2000 Jan 74(2):619-626.

Tsai et al. Cell. 2002 Aug 9;110(3),373-83.

Watt et al. Science. 2000; 287; 1427-1430.

Weissman Science. 2000; 287;1442-1446.

Wilson et al. Drug Des Discov. 2000;17(2):105-14.

Young Science. 2000;287;1424.

Jellinger K. the pathology of parkinsonism. In movement disorders 2 (Marsden, C.D. and Fahn, S., eds), pp.124-165, Butterworth-Heineman.

*THE* α-*CHEMOKINES CXCL11, 12 AND 13 REGULATE THE PROLIFERATION OF VENTRAL MIDBRAIN PRECURSORS AND THEIR DIFFERENTIATION INTO DOPAMINERGIC NEURONS*

c17.2 neural stem cells stably transfected with Nurr1 showed increased expression of the chemokine orphan receptor Cmkor1 (RDC1). This receptor belongs to the CXCR family of G-protein-coupled seven-transmembrane-spanning receptors and was first identified as a co-receptor for HIV-1 (Shimizu et al., 2000). Chromosomal mapping of the mouse locus for Cmkor1/RDC1 between the genes of two characterized chemokine receptors, CXCR2 and CXCR4, and the fact that its closest similarity is to CXCR2 among all the G-protein-coupled seven-pass transmembrane receptors, suggest that chemokines are potential ligands for RDC1 (Heesen et al., 1998). CXCR2 has been reported to control the development and positioning of oligodendrocyte precursors in the developing spinal cord (Tsai et al., 2002). More recently, CXCL12/SDF1 and CXCR4 have been identified as the ligand and co-receptor of RDC1, respectively (Balabanian et al., 2005). A recent report has shown that CXCL12/SDF1 and CXCR4 receptors play an important role in the initial guiding of motor neuron axons (Lieberam et al., 2005). However, it is not know whether CXCL12 and its receptors CXCR4 and RDC1 regulate any other aspect of neuronal development.

In this study we characterize the expression of CXCL12 and identify two other α-chemokines, CXCL11 and CXCL13, and their receptors, RDC1, CXCR3, 4 and 5, that are also expressed in the developing ventral midbrain. Moreover, we found that administration of CXCL11, 12 or 13 to ventral midbrain precursor cultures promotes proliferation of VM precursors and their differentiation into tyrosine hydroxylase (TH)+/Nurr1+ neurons. We also identify RDC1, one of the receptors for CXCL12, as a critical signaling component since RDC1 RNAi very significantly reduced neurogenesis in Nurr1+ cells. In sum, our study identifies several α-chemokines as critical regulators of VM development. Moreover our functional data, together with the high levels of expression of CXCL12, and their receptors CXCR4 and RDC1, suggest that the this ligand and receptors are key regulators of dopaminergic neurogenesis.

### MATERIAL AND METHODS

### Real time RT-PCR and quantification of gene expression

Total RNA was isolated from ventral midbrain of mice embryonic day (E) 10.5, 11.5, 13.5, 15.5 and P1. Reverse transcription reaction and real time RT-PCR was carried out as described (Castelo-Branco et al. 2003). Quantum RNA classical 18S internal standard was purchased from Ambion (Austin, USA) and PCR primers from DNA Technology a/S Aarhus, Denmark. The following PCR programme was used for SYBR Green detection on the ABI PRISM 5700 Detection System (PE Applied Biosystems, Foster City, CA, USA): 94°C for 2 minutes; 35-40 cycles of 94°C for 30 seconds, 60°C for 30 seconds, 72°C for 15 seconds; and 80°C for 5 seconds. Statistical analysis of the results was performed using a two-tailed Wilcoxson Signed rank test: Significance for all tests was assumed at the level of P<0.05(*P<0.05, **0.01<P<0.001; ***P<0.001).

### Precursor cultures and treatments

VM from 14.5 embryos obtained from time-mated Sprague-Dawley rats, were dissected, mechanically dissociated, and plated at a final density of 1x10⁵ cells/cm² on poly-D lysine coated 6- or 24-well plates in N2 serum free medium, consisting of a 1:1 mixture of F12 and MEM with N2 supplement, 300mg glucose, 50mg BSA, 750microliter HEPES and 250 microliter glutamine. All factors were added once, at the initiation of culture and BrdU was added 6 hours prior to fixation.

### Immunocytochemical analysis

Cells were fixed for immunocytochemistry in ice-cold 4% paraformaldehyde for 15-20 minutes and washed with PBS and incubated over night in dilution buffer (1xPBS 1% BSA, and 0.3% Triton-X 100) with one of the following antibodies: rat ∝BrdU (1:200; Abcam), Tuj1 (1:500; Abcam). After washing, cultures were incubated for1-2 hours in dilution buffer with the appropriate secondary antibody/ies (biotinilated 1:500, CY2 or rhodamine coupled horse-anti-mouse IgG 1:100, all from vector). Bright field immunostaining was visualized with the Vector Laboratory ABC immunoperoxidase kit, using NovaRed (red), SG or 3-3' diaminobenzidine tetrahydrocloride (DAB 0.5 mg/ml)/nickel chloride (1.6 mg/ml) (gray/black), or VIP (violet) substrates. Double immunostaining was performed by sequential single staining as described above. At the end of all staining procedures, cultures were incubated with Hoechst 33258 reagent for 10 minutes. BrdU immunocytochemistry included incubation for 30 minutes with 2M HCL. Images were acquired from stained cells in PBS at room temperature with a Zeiss Axioplan 100M microscope (LD Achrostigmat 20X, 0.3 PHI ∝0.2 and LD Achroplan 40X, 0.60 Korr PH2 ∝0.2) and collected with a Hamamatsu camera C4742.95 (with QED imaging software).

### RESULTS:

### α-chemokines are expressed in the developing ventral midbrain at the time of DA neurogenesis

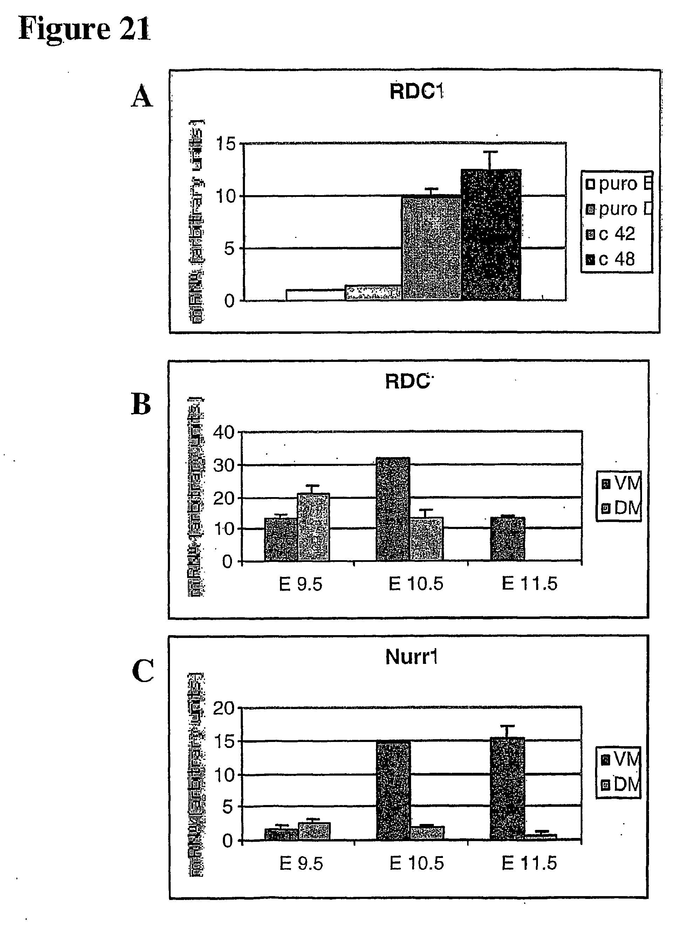

We have previously found that the Cmkor1/RDC1 receptor is expressed at high levels in a Nurr1-overexpressing neural stem cell line (Sousa et al., in preparation). To confirm that result, we compared independent cultures of the same cell lines by real-time RT-PCR analysis, and we found that Cmkor1/RDC1 is induced around 9-fold in the Nurr1 transfected clones (Figure 21A). Consistent with a role of Nurr1 in regulating RDC1 expression, we found that RDC1 is expressed at high levels in the VM and that its expression levels peak at E10.5 (Figure 21B), coinciding with the onset of Nurr1 expression (Figure 21C).

To investigate the role of chemokines during midbrain morphogenesis in more detail, we performed a genechip analysis using total mRNA extracted from mouse embryonic ventral midbrain (VM) and dorsal midbrain (DM) tissue at two developmental stages, E10.5 and E12.5. The mRNA was retrotranscribed to ³²P-labelled cDNA and was hybridized to a GEArray membrane containing 67 chemokines and chemokine receptor genes. Out of the genes that we found to be expressed in the developing midbrain (Table 4), we focused our attention on the ligands and receptors belonging to the CXC subfamily. In the E10.5 midbrain, three alpha chemokine receptors (CXCRS/Blr1, CXCR4/Cmkar4 and RDC1/Cmkor1) and three CXC ligands (CXCL11/I-TAC, CXCL15/Scyb15 and SDF-1) are expressed at higher levels in the VM, compared to the DM. These same genes were also expressed at a later stage, at E12.5, in the VM.

### α-chemokines promote the proliferation of ventral midbrain precursors and their differentiation into DA neurons

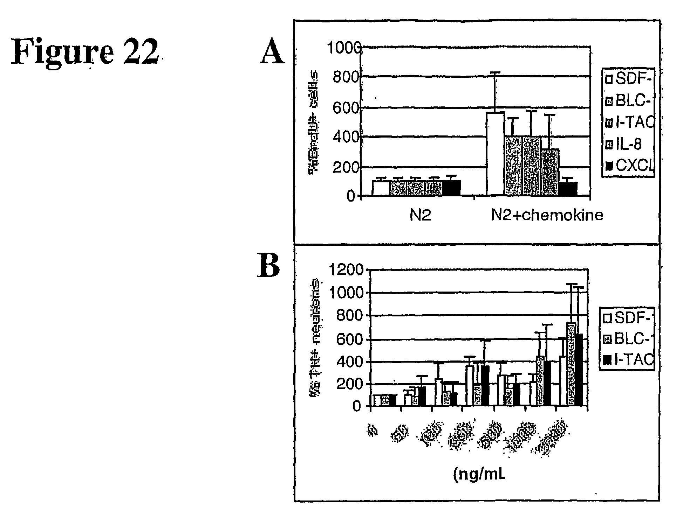

To elucidate the role of these genes in dopaminergic development, we treated rat E14.5 VM primary precursor cultures with several CXCL ligands. High doses of the CXCL13/BLC-1, a ligand that binds CXCR5, and CXCL12/SDF-1, a ligand that binds specifically to CXCR4 and RDC1, promoted proliferation (Figure 22A) and increased the number of TH+ neurons in the cultures (Figure 22B). CXCL11/I-TAC, a ligand for CXCR3, had a similar effect (Figure 22A and 21B).

### DISCUSSION

Our results indicate that overexpression of Nurr1 in a neural stem cell promotes the acquisition of a region specific neuronal cell fate by: 1) Downregulating genes involved in maintenance of the stem cell state, proliferation, and differentiation into other cell types; 2) Upregulating genes involved in providing regional identity and in promoting neuronal differentiation and survival. Moreover, Nurr1 regulated the expression of the Cmkor1/RDC1, and Cmkor1/RDC1 and its coreceptor CXCR4 and ligand CXCL12/SDF1 were the only receptor-ligand pair that were expressed at high levels in the VM. Other receptors expressed at high levels in the VM included the CXCR5/Blr1 receptor, Ltb4r2 and Tapbp. Other ligands also expressed at high levels included CXCL11/I-TAC, CXCL15 and SDF-2. Interestingly SDF1/CXCL12, the ligand of RDC1 and the CXCR4 receptors, regulated the proliferation and the numbers of TH+ neurons in VM primary precursor cultures. We hereby report that other α-chemokines, including CXCL13/BLC-1 (that binds CXCR5/Blr1), and CXCL11/I-TAC (that binds CXCR3) also regulated precursor proliferation and dopaminergic differentiation in a similar manner. We also have previously shown that CXCL8 exerted similar functions, and that CXCL6 promoted the maturation of dopaminergic precursors into neurite-rich dopaminergic neurons (Edman et al., unpublished). All these results show that multiple □-chemokines are not only present, but are also capable of regulating diverse aspects of dopaminergic development. Our results point to SDF1/CXCL12 and the RDC1 and CXCR4 receptors as one of the main target genes regulated by Nurr1 since it is regulated in our genechip experiment. Moreover, RDC1 expression peaks shortly after the expression of Nurr1 during VM development suggesting that Nurr1 may also regulate RDC1 expression in vivo. We have previously proposed that one of the main functions of Nurr1 is to induce the expression of a receptor or signaling component conferring Nurr1-expressing cells the capacity to respond to a signal derived from glial cells (Hall et al., 2003). The present results are consistent with this possibility since Nurr1+ cells express RDC1 and glial cells are known to express chemokines (Lazarini et al., 2003; Bajetto et al., 2002). Moreover, we found that SDF1/CXCL12 promote the proliferation and differentiation of precursors into midbrain DA neurons. Furthermore, RDC1 RNA interference experiments showed a dose-dependent blockade reaching a near complete loss of neurogenesis in Nurr1-expresing neural stem cells. Thus, our results indicate that SDF1/CXCL12 and the RDC1 and CXCR4 receptors play an essential role in dopaminergic neuron development. Our data also provide indication for α-chemokines in a potential therapeutic application by promoting stem/precursor cell differentiation into dopaminergic neurons. These cells may be used for drug discovery/toxicity assays in dopaminergic neurons or for cell replacement therapy in Parkinson's disease patients. Another therapeutic avenue is the application of small molecules that activate chemokine receptors to either promote or contribute to endogenous adult dopaminergic neurogenesis in patients of Parkinson's disease.

**TABLE 1**

| Genes upregulated in c17.2-Nurr1 stem cells | | | | |
|---|---|---|---|---|
| **Protein** | **Function** | **Expression ratio** | | **Accession no.** |
| | | **c42/pB** | **c48/pD** | |
| *Extracellular matrix*/*cell structure* | | | | |
| Tenascin C | differentiation | 17.5 | 3.7 | AV230686 |
| H2A/H2B histones | chromatin | 8.2 | 2.5 | X05862 |
| β tubulin III | cytoskeleton | 4.0 | 3.4 | AV350617 |
| Neurofilameat, medium polypeptide | cytoskeleton | 3.5 | 3.0 | X05640 |
| H1 histone | chromatin | 2.7 | 2.4 | J03482 |

| *Transcription* | | | | |
|---|---|---|---|---|
| LRG-21/ATF3 | stress response | 2.5 | 2.4 | U19118 |
| Engrailed-1 | homedomain protein | 2.4 | 2.4 | L12703 |
| Runx2 | differentiation | 2.1 | 2.5 | D14636 |
| Slug | differentiation | 2.0 | 2.1 | U79550 |
| Peg1/MES T | imprinting | 1.6 | 23.6 | AF017994 |

| *Secreted proteins* | | | | |
|---|---|---|---|---|
| Small inducible cytokine A7 (MCP-3) | chemokine | 20.7 | 1.5 | X70058 |
| Nerve growth factor, beta | neurotrophin | 5.4 | 2.5 | M17298 |
| Small inducible cytokine A2 (MCP-1) | chemokine | 4.3 | 1.4 | M19681 |
| IGFBPS endopeptidase | serine protease | 3.3 | 2.4 | AW125478 |
| Stanuiocalcin (Stc2) | calcium homeostasis | 2.6 | 2.3 | AF031035 |
| Wnt1 inducible secreted protein 1 | growth factor | 2.1 | 2.7 | AF100777 |
| TGF-β | growth factor | 2.0 | 2.0 | L19932 |

| *Other* | | | | |
|---|---|---|---|---|
| Interferon inducible gene 202 | survival | 5.6 | 1.6 | M31418 |
| ATP-binding cassette 1 | cholesterol efflux | 3.2 | 2.6 | AI845514 |
| Unknown | unknown | 3.2 | 1.4 | AW125508 |
| Unknown | unknown | 2.1 | 2.7 | AI844196 |

**TABLE 2**

| Genes downregulated in c17.2-Nurr1 stem cells | | | | |
|---|---|---|---|---|
| **Protei n** | **Function** | **Expression ratio** | | **Accession no.** |
| | | **c42/pB** | **c48/pD** | |
| *Extracellular matrix*/*cell structure* | | | | |
| Coagulation factor III | coagulation | -4.7 | -2.0 | M26071 |
| Integrin α4 | cell adhesion | -4.4 | -2.6 | X53176 |
| Procollagen, type IV, α1 | cell adhesion | -3.6 | -1.8 | M15832 |
| β-filamin | cytoskeleton/migration | -2.3 | -2.0 | AI838592 |
| Myosin X | cytoskeleton | -2.5 | -2.4 | AJ249706 |

| *Transcription*/*Signaling* | | | | |
|---|---|---|---|---|
| ADP ribosyltransferase 3 | protein inactivation | -3.7 | -1.7 | Y08027 |
| Pr13 | tyrosine phosphatase | -3.3 | -1.7 | AF035645 |
| Brain fatty acid binding prote i n | neural stem cell marker | -2.7 | -6.4 | U04827 |
| Caspase-11 | apoptosis | -2.7 | -2.2 | Y13089 |
| Ran GTPase activating protein-1 | cytoskeleton | -2.4 | -2.2 | U20857 |
| Caspase-3 | apoptosis | -2.4 | -1.8 | U54803 |
| ASM-like phosphodiesterase 3a | ceramide release | -1.9 | -4.4 | Y08135 |
| Eps8 | cytoskeleton | -1.8 | -4.5 | L21671 |
| Myeloblastosis oncogene-like 1 | chromatin | -1.5 | -3.5 | L35261 |

| *Other* | | | | |
|---|---|---|---|---|
| Unknown | unknown | -2.2 | -2.3 | AA797843 |
| Zygin I | membrane fusion | -1.6 | -5.0 | AI841076 |
| Unknown | unknown | -1.3 | -4.1 | AW121304 |

**TABLE 3**

| Validation of microarray genes by quantitative PCR | | | | |
|---|---|---|---|---|
| | **c42/pB** | | **c48/pD** | |
| | **Affymetrix** | **RT-PCR** | **Affymetrix** | **RT-PCR** |
| *Secreted proteins* | | | | |
| WISP 1 | 2.1 | 2 | 2.7 | 3.7 |
| IGFBP5 endopeptidase | 33 | 9.1 | 2.4 | 5.4 |
| Small inducible cytokine A2 | 43 | 3.1 | 1.4 | 1.9 |
| Small inducbile cytokine A7 | 20.7 | 4.6 | 1.5 | 2.2 |

| *Cell structure* | | | | |
|---|---|---|---|---|
| Neurofilament, medium peptide | 3.5 | 1.2 | 3.0 | 5.5 |
| H2A/H2B histones | 8.2 | - 4 | 2.5 | 1.3 |

| *Signaling* | | | | |
|---|---|---|---|---|
| Caspase-11 | -2.7 | -1.5 | -2.2 | -1.7 |
| Caspase-3 | -2.4 | -2.6 | -1.8 | -2.3 |

| *Transcription* | | | | |
|---|---|---|---|---|
| LRG-21 / ATF3 | 2.5 | 9.1 | 2.4 | 2 |
| Engrailed 1 | 2.4 | 4.5 | 2.4 | 1.0 |
| Slug | 2.0 | 3.2 | 2.1 | 2.5 |

**TABLE 4**

| Chemokine qene expression profile of mouse E10.5 midbrain | | |
|---|---|---|
| Gene Group | Detected | Not detected |
| *Small inducible* | | |
| *cytokines* | | |
| Subfamily A | Scya6 (C10), | Scya1 (TCA3), Scya2 (JE), |
| (Cys-Cys) | Scya7 (MARC) | Scya3 (MIP-1α), Scya4 (MIP-1β), |
| | | Scya5 (RANTES), Scya8 (MCP-2), |
| | | Scya9 (MRP-2), Scya11 (eotaxin), |
| | | Scya12 (mcp-5), Scya17 (TARC), |
| | | Scya19 (MIP-3β), Scya20 (MIP-3α), |
| | | Scya21a (SLC), Scya22(MDC), |
| | | Scya24 (CCL24), Scya25 (TECK), |
| | | Scya27 (ALP), Scya28 (MEC) |
| Subfamily B | Scyb11 (CXCL11/ | CXCL16 (SR-PSOX), Gro1, |
| (Cys-X-Cys) | I-TAC), | Scyb2 (MIP2), Scyb5 (CXCL-6/GCP- |
| | Scyb15 (CXCL15) | 2/ENA78), Scyb9(Mig), Scyb10 (IP- |
| | , Sdf-1α | 10), Scyb13 (BLC), |
| | | Scyb14 (Kec/Bmap) |
| *Chemokine receptors* | Blr1, | Ccxcr1 (mXCR1), Cmkar3 (CXCR3), |
| | Cmkar4 (CXCR4), | Cmkbr1 (CCR-1), Cmkbr1I1, |
| | Cmkor1 (RDC1), | Cmkbr!I2, Cmkbr2, Cmkbr3, Cmkbr4, |
| | Ltb4r2, Tapbp | Cmkbr5, Cmkbr6(CCR6), |
| | | Cmkbr7 (EBI1), Cmkbr8 (CCR8), |
| | | Cmkbr9(GPR2), Cmkbr10, Cx3cr1, |
| | | Cxcr6, D6-pending, IL8rb (Cmkar2), |
| | | Pumag |
| *Other Related Genes* | Emap2, Epo, | Dfy, Ifna11, Pf4, Ppbp, human PRL |
| | Ifnab, Sdf2, | homolog, Tcp10 |
| | 5830453M24Rik | |

### Additional Statements of invention

1. A method of promoting survival, differentiation and/or maturation of a neural stem cell, precursor or progenitor cell, or a neuronal cell, comprising
   (a) modulating, optionally increasing, activity of a protein listed in Table 1 in the cell; and/or
   (b) modulating, optionally decreasing activity of a protein listed in Table 2 in the cell;
   (c) modulating activity of one or more of tenascin-C; an α-chemokine, optionally an α-chemokine selected from the group consisting of CXCL6, CXCL8, CXCL11, CXCL12 and CXCL13; a receptor for an α-chemokine, optionally an α-chemokine selected from the group consisting of CXCR2, CXCR3, CXCR4, CXCR5; RDC1; a β-chemokine, optionally a β-chemokine, selected from the group consisting of CCL2 and CCL7; and a receptor for a β-chemokine, optionally CCR2;
   wherein the method does not consist of the following:
   increasing Nurr1 activity in the cell, contacting the cell with one or more factors obtainable from a Type 1 astrocyte of the ventral midbrain and/or contacting the cell with a Wnt polypeptide.
2. A method according to 1, wherein step (a) comprises increasing an activity of one or more proteins listed in Table 3.
3. A method according to 2, comprising increasing RDC1 activity.
4. A method according to any one of the preceding claims,
   wherein protein activity is increased by introducing nucleic acid encoding the protein into the cell, and causing or allowing the nucleic acid to be expressed in the cell.
5. A method according to any one of 1 to 4
   wherein protein activity is increased by introducing the protein into the cell.
6. A method according to any one of 1 to 4
   wherein protein activity is decreased using RNA interference or antisense.
7. A method according to 5 wherein the method comprises contacting the cell with a modulator, wherein the modulator
   (a) modulates, optionally increases activity of a one or more proteins listed in Table 1;
   (b) modulates, optionally decreases activity of one or more proteins listed in Table 2; and/or
   (c) modulates activity of one or more of tenascin-C; an α-chemokine, optionally an α-chemokine selected from the group consisting of CXCL6, CXCL8, CXCL11, CXCL12 and CXCL13; a receptor for an α-chemokine, optionally an α-chemokine selected from the group consisting of CXCR2, CXCR3, CXCR4, CXCR5; RDC1; a β-chemokine, optionally a β-chemokine selected from the group consisting of CCL2 and CCL7; and a receptor for a β-chemokine, optionally CCR2.
8. A method of screening for a substance able to modulate an activity of a protein encoded by a Nurr1 regulated gene, including:
   (a) contacting a cell expressing DNA containing the promoter of said gene with a test substance, wherein the promoter is operably linked to a gene
   (b) determining the level of gene expression from the promoter, and
   (c) comparing the level of gene expression in the presence of the test substance with the level of gene expression in the absence of the test substance in comparable conditions,
   wherein a difference in the level of gene expression indicates that the test substance is able to modulate expression of the gene.
9. A method according to 8, wherein the promoter is operably linked to a heterologous gene.
10. A method according to 9, wherein the heterologous gene is a reporter gene.
11. A method of screening for a substance able to modulate activity of a protein encoded by a Nurr1 regulated gene, including:
   (a) bringing into contact the protein and a test substance;
   (b) determining the protein activity, and
   (c) comparing the protein activity in the presence of the test substance with the protein activity in the absence of the test substance in comparable reaction medium and conditions,
   wherein a difference in protein activity indicates that the test substance is able to modulate the protein activity.
12. A method according to any one of 8 to 11 further comprising identifying the test substance as a modulator of activity of a protein encoded by a Nurr1-regulated gene.
13. A method according to 12 further comprising purifying or isolating the test substance.
14. A method according to 12 or 13 further comprising developing the test substance to obtain a mimetic.
15. A method of promoting cell proliferation and increasing the number of tyrosine hydroxylase-expressing cells in a group of neural cells, comprising contacting the cells with one or more chemokines selected from the group consisting of an α-chemokine, optionally an α-chemokine selected from the group consisting of CXCL6, CXCL8, CXCL11, CXCL12 and CXCL13; a β-chemokine, optionally a β-chemokine selected from the group consisting of CCL2 and CCL7, CCR2BLC-1, SDF1 and IL-8,
16. A method according to 15, wherein the cells are a mixed primary culture.
17. A method according to 15 or 16, wherein the cells are ventral mesencephalic cells.
18. A method according to 15, wherein the cells are stem or neural precursor or progenitor cells.
19. A method of promoting the differentiation and/or maturation of a stem, neural stem, precursor or progenitor cell into a neurone, comprising contacting the cell with CXCL12.
20. A method of promoting neuritogenesis in a precursor, progenitor or neuronal cell, comprising contacting the cell with CXCL12.
21. A method according to any one of 1 to 7 or 15 to 20, further comprising contacting the cell with one or more Wnt polypeptides.
22. A method according to 21, comprising contacting the cell with Wnt1.
23. A method according to 21 or 22, comprising contacting the cell with Wnt5a.
24. A method according to any one of 21 to 23, comprising contacting the cell with Wnt3a.
25. A method according to any one of 1 to 7 or 15 to 24, wherein the method is performed in *vitro* or *ex vivo.*
26. A method according to any one of 1 to 7 or 15 to 24, wherein the method is performed *in vivo.*
27. A method according to 26 wherein the method is performed *in situ* in the brain of an individual.
28. An isolated neural cell treated according to a method of claim 25, or a descendent of a cell so treated.
29. An isolated neural cell according to 28, which contains exogenous nucleic acid.
30. An isolated neural cell according to 28 or 29, which is a neurone.
31. An isolated neural cell according to any one of 28 to 30, for use in a method of medical treatment.
32. An isolated neural cell according to 31, for use in treating neurodegenerative disease or neuronal loss.
33. An isolated neural cell according to 32, wherein the disease is Parkinson's disease or parkinsonian syndrome.
34. A substance selected from the group consisting of:
   nucleic acid encoding a Nurr1-upregulated gene; a protein encoded by a Nurr1-upregulated gene; double stranded RNA encoding a Nurr-1 downregulated gene; nucleic acid complementary to a sequence encoding a Nurr-1 downregulated gene; a BLC-1, SDF1, IL-8, α-chemokine, optionally an α-chemokine selected from the group consisting of CXCL6, CXCL8, CXCL11, CXCL12 and CXCL13, a β-chemokine, optionally a β-chemokine selected from the group consisting of CCL2 and CCL7, or nucleic acid encoding such a chemokine, for use in a method of medical treatment.
35. A substance according to 34, for use in treating neurodegenerative disease or neuronal loss.
36. A substance according to 35, wherein the disease is Parkinson's disease or parkinsonian syndrome.
37. Use of an isolated neural cell according to any one of
   28 to 30 and/or a substance selected from the group consisting of: nucleic acid encoding a Nurr1-upregulated gene; a protein encoded by a Nurr1-upregulated gene; double stranded RNA encoding a Nurr-1 downregulated gene; nucleic acid complementary to a sequence encoding a Nurr-1 downregulated gene; a BLC-1, SDF1, IL-8, α-chemokine, optionally an α-chemokine selected from the group consisting of CXCL6, CXCL8, CXCL11, CXCL12 and CXCL13, a β-chemokine, optionally a β-chemokine selected from the group consisting of CCL2 and CCL7, and nucleic acid encoding such a chemokine, in the manufacture of a medicament for treating neurodegenerative disease or neuronal loss.
38. Use according to 37 wherein the medicament is for administration to the brain of an individual.
39. Use according to 37 or 38 wherein the disease is Parkinson's disease or parkinsonian syndrome.
40. A method of manufacturing a medicament for treating neurodegenerative disease or neuronal loss, comprising:
   (a) treating a neural cell using a method according to 25; and
   (b) formulating the neural cell, or a descendent of the cell, into a composition comprising a pharmacologically acceptable excipient.
41. A method according to 40, wherein the medicament is for implantation into the brain of an individual.
42. A method of treating neurodegenerative disease or neuronal loss in an individual, comprising
   (a) treating a neural cell using a method according to 25; and
   (b) administering the neural cell, or a descendent *of* the cell, to the individual.
43. A method of treating neurodegenerative disease or neuronal loss in an individual, comprising administering a neural cell according to any one of 28 to 30 to the individual.
44. A method according to 42 or 43, wherein the cell is endogenous to the individual.
45. A method according to 42 or 43, wherein the cell is exogenous to the individual.
46. A method according to any one of 42 to 45,
   wherein administering comprises implanting the cell into the brain of the individual.
47. A method of treating a neurodegenerative disease or neuronal loss in an individual, comprising administering to the individual a substance selected from the group consisting of: nucleic acid encoding a Nurr1-upregulated gene; a protein encoded by a Nurr1-upregulated gene; double stranded RNA encoding a Nurr-1 downregulated gene; nucleic acid complementary to a sequence encoding a Nurr-1 downregulated gene; a BLC-1, SDF1, IL-8, α-chemokine, optionally an α-chemokine selected from the group consisting of CXCL6, CXCL8, CXCL11, CXCL12 and CXCL13, a β-chemokine, optionally a β-chemokine selected from the group consisting of CCL2 and CCL7 chemokine and nucleic acid encoding such a chemokine.
48. A method of treating a neurodegenerative disease in an individual, comprising treating a neural cell using a method according to any one of 1 to 7 or 15 to 24, wherein the neural cell is *in situ* in brain of the individual.
49. A method according to 48, wherein the cell is endogenous to the individual.
50. A method according to 49, wherein the cell is exogenous to the individual.
51. A method according to any one of 42 to 50,
   wherein the neurodegenerative disease is Parkinson's disease or parkinsonian syndrome.
52. Use of a cell of the present invention in toxicology screening and/or drug discovery.
53. A method of determining toxicity of a substance or of identifying a substance as a drug candidate, comprising treating a cell of the present invention with a test substance and determining the effect of the test substance on the cell, or viability, survival, proliferation, differentiation or maturation, and/or one or more properties or phenotypes of the cell.

## Claims

1. A method of increasing the number of tyrosine hydroxylase-expressing cells in a group of neural cells, comprising contacting the cells *in vitro* or *ex vivo* with one or more chemokines selected from the group consisting of an α-chemokine, optionally an α-chemokine selected from the group consisting of CXCL12, CXCL8, CXCL6, CXCL11, and CXCL13; a β-chemokine, optionally a β-chemokine selected from the group consisting of CCL2 and CCL7, and CCR2BLC-1.

2. A method according to claim 1, wherein the cells are stem or neural precursor or progenitor cells.

3. A method according to claim 1 or claim 2, wherein the cells are ventral mesencephalic cells.

4. A method according to claim 1, wherein the cells are a mixed primary culture.

5. A method of promoting the proliferation, differentiation and/or maturation of a stem, neural stem, precursor or progenitor cell into a neurone, comprising contacting the cell *in vitro* or *ex vivo* with one or more of an α-chemokine, optionally an α-chemokine selected from the group consisting of CXCL12, CXCL8, CXCL6, CXCL11, and CXCL13 and a β-chemokine, optionally a β-chemokine selected from the group consisting of CCL2 and CCL7.

6. A method of promoting neuritogenesis in a precursor, progenitor or neuronal cell, comprising contacting the cell *in vitro* or *ex vivo* with CXCL12, CCL2 or CCL7.

7. A method of promoting survival, proliferation, neurogenesis, differentiation and/or maturation of a neural stem cell, precursor or progenitor cell, or a neuronal cell, comprising modulating activity in the cell *in vitro* or *ex vivo* of one or more of an α-chemokine, optionally an α-chemokine selected from the group consisting of CXCL12, CXCL8, CXCL6, CXCL11, and CXCL13; a receptor for an α-chemokine, optionally an α-chemokine receptor selected from the group consisting of RDC1; CXCR4, CXCR2, CXCR3, CXCR5; a β-chemokine, optionally a β-chemokine selected from the group consisting of CCL2 and CCL7; and a receptor for a β-chemokine, optionally CCR2.

8. A method according to claim 7, wherein protein activity is increased by introducing nucleic acid encoding the protein into the cell, and causing or allowing the nucleic acid to be expressed in the cell.

9. A method according to claim 7 or claim 8, wherein protein activity is increased by introducing the protein into the cell.

10. A method according to any one of claims 7 to 9, wherein protein activity is decreased using RNA interference or antisense.

11. A method according to claim 9 wherein the method comprises contacting the cell with a modulator, wherein the modulator modulates activity of one or more of an α-chemokine, optionally an α-chemokine selected from the group consisting of CXCL12, CXCL8, CXCL6, CXCL11, and CXCL13; a receptor for an α-chemokine, optionally an α-chemokine receptor selected from the group consisting of RDC1, CXCR4, CXCR2, CXCR3 and CXCR5; a β-chemokine, optionally a β-chemokine selected from the group consisting of CCL2 and CCL7; and a receptor for a β-chemokine, optionally CCR2.

12. A method according to any one of claims 1 to 11, further comprising contacting the cell with one or more Wnt polypeptides.

13. A method according to claim 12, comprising contacting the cell with Wnt5a.

14. A method according to claim 12 or claim 13, comprising contacting the cell with Wnt1.

15. A method according to any one of claims 12 to 14, comprising contacting the cell with Wnt3a.

16. A substance selected from the group consisting of: an α-chemokine, optionally an α-chemokine selected from the group consisting of CXCL12, CXCL8, CXCL6, CXCL11, and CXCL13, a β-chemokine, optionally a β-chemokine selected from the group consisting of CCL2 and CCL7, or nucleic acid encoding such a chemokine, for use in a method for treating neurodegenerative disease or neuronal loss.

17. A substance according to claim 16, wherein the disease is Parkinson's disease or parkinsonian syndrome.

18. Use of an isolated neural cell and/or a substance selected from the group consisting of: an α-chemokine, optionally an α-chemokine selected from the group consisting of CXCL12, CXCL8, CXCL6, CXCL11, and CXCL13, a β-chemokine, optionally a β-chemokine selected from the group consisting of CCL2 and CCL7, and nucleic acid encoding such a chemokine, in the manufacture of a medicament for treating neurodegenerative disease or neuronal loss, wherein for said isolated neural cell said manufacture comprises treating a neural cell in accordance with the method of any one of claims 1-15.

19. Use according to claim 18 wherein the medicament is for administration to the brain of an individual.

20. Use according to claim 18 or claim 19 wherein the disease is Parkinson's disease or parkinsonian syndrome.

21. A method of manufacturing a medicament for treating neurodegenerative disease or neuronal loss, comprising:
(a) treating a neural cell using a method according to any one of claims 1-15; and
(b) formulating the neural cell, or a descendent of the cell, into a composition comprising a pharmacologically acceptable excipient.

22. A method according to claim 21, wherein the medicament is for implantation into the brain of an individual.
